# NEUE EUROPÄISCHE PATENTSCHRIFT

(11) **EP 0 888 386 B2**
(45) Veröffentlichungstag und Bekanntmachung des Hinweises auf die Entscheidung über den Einspruch: **01.04.2009**
(45) Hinweis auf die Patenterteilung: 06.11.2002
(21) Anmeldenummer: 97906928.3
(22) Anmeldetag: 13.03.1997
(51) Int. Cl.: C07K 14/755, A61K 38/37

(54) **Stabiles, virussicheres Faktor VIII-Komplex Konzentrat**
Stable, virus-safe factor VIII-complex concentrate
Concentré d'un complèxe du facteur VIII, stable et sans virus

(30) Priorität: 15.03.1996 AT 49496
(43) Veröffentlichungstag der Anmeldung: 07.01.1999
(73) Patentinhaber: Baxter Aktiengesellschaft, 1221 Wien (AT)
(72) Erfinder: FISCHER, Bernhard, A-1120 Wien (AT); MITTERER, Artur, A-2304 Mannsdorf 116 (AT); DORNER, Friedrich, A-1230 Wien (AT); EIBL, Johann, A-1180 Wien (AT)
(74) Vertreter: Alge, Daniel
(86) Internationale Anmeldenummer: PCT/AT1997/000055
(87) Internationale Veröffentlichungsnummer: WO 1997/034930

(56) Entgegenhaltungen:
- EP-A- 0 600 480
- EP-A- 0 705 846
- WO-A-96/10584
- DE-A- 3 504 385
- US-A- 5 110 907
- US-A- 5 149 787
- THROMBOSIS RESEARCH 84 (1). 1996. 55-66, XP000674503 FISCHER B E ET AL: "Effect of multimerization of human and recombinant von Willebrand factor on platelet aggregation, binding to collagen and binding of coagulation factor VIII."
- BURNOUF-RADOSEVICH M.; BURNOUF T.: 'Vox Sang', Bd. 62, 1962, S. KARGER AG, BASEL Seiten 1 - 11
- 'Centeon Codex', Januar 1996 Artikel 'Fachinformation über Haemate HS', Seiten 25 - 30

## Beschreibung

Die Erfindung betrifft einen stabilen virussicheren Faktor VIII-Komplex, der insbesondere hochmolekulare vWF-Multimere mit hoher struktureller Integrität enthält. Desweiteren betrifft die Erfindung Verfahren zur Gewinnung und Herstellung eines stabilen Faktor VIII-Komplexes sowie pharmazeutische Präparationen davon.

Die Blutgerinnung ist ein komplexer Prozeß, der die sequentielle Interaktion einer Reihe von Komponenten, insbesondere von Fibrinogen, Faktor II, Faktor V, Faktor VII, Faktor VIII, Faktor IX, Faktor X, Faktor XI und Faktor XII einschließt. Der Verlust einer dieser Komponenten oder die Inhibierung ihrer Funktionalität führt zu einer verstärkten Blutgerinnungsneigung, die bei einigen Patienten lebensbedrohlich sein kann.

Von Willebrand-Faktor (vWF) zirkuliert im Plasma komplexiert mit Faktor VIII, wobei Faktor VIII die Blutgerinnung unterstützt und vWF im Komplex mit Faktor VIII diesen stabilisiert und vor proteolytischem Abbau schützt. Durch seine Funktion bei der Plättchenaggregation greift vWF auch direkt in die Blutgerinnung ein. Der vWF ist ein Glykoprotein, welches in verschiedenen Zellen von Säugern gebildet und anschließend in die Zirkulation freigesetzt wird. Dabei wird in den Zellen ausgehend von einer Polypeptidkette mit einem Molekulargewicht von ca. 220 kD durch Ausbildung von mehreren Schwefelbrücken ein vWF-Dimer mit einem Molekulargewicht von 550 kD gebildet. Aus den vWF-Dimeren werden dann durch Verknüpfung weitere Polymere des vWF mit immer höherem Molekulargewicht, bis zu 20 Millionen Dalton, hergestellt. vWF existiert daher im Plasma in einer Serie von multimeren Formen von einem Molekulargewicht von 1 x 10⁶ bis 20 x 10⁶ Dalton. Es wird vermutet, daß insbesondere den hochmolekularen vWF-Multimeren eine essentielle Bedeutung bei der Blutgerinnung zukommt.

Neben der Carrier-Funktion für Gerinnungsfaktor VIII besteht für den vWF die Funktion in der Brücken-Bildung zwischen Gefäßwand und der Plättchen und Plättchenagglutination. Die Grundlage zur Plättchenagglutination ist durch die Bindung des vWF an Oberflächen-Rezeptoren (Glykoproteine Ib, IIb/IIIa) gegeben. Die Bindungsstelle innerhalb des vWF zur Bindung an GP Ib befindet sich im Disulfid-Loop Cys(509)-Cys(695). Es ist bekannt, daß die Plättchenagglutination mit einer Bindung des vWF an das Glykoprotein Ib beginnt. Nach einem Aktivierungssignal kommt es dann zur Bindung des vWF an den Glykoprotein IIb/IIIa-Komplex und der Agglutination. Die Plättchenagglutination setzt somit die Bindung des vWF an die Oberflächenrezeptoren voraus; durch die Bindung mehrerer Plättchen durch ein vWF-Molekül kommt es in Folge zur Agglutination. vWF-Plättchenbindung stellt somit die molekulare Ursache fUr die Plättchenagglutination dar.

Bei der Hämophilie ist die Blutgerinnung durch Mangel an bestimmten plasmatischen Blutgerinnungsfaktoren gestört. Bei der Hämophilie A beruht die Blutungsneigung auf einem Mangel an Faktor VIII bzw. einem Mangel an vWF, der ein wesentlicher Bestandteil des Faktors VIII darstellt. Die Behandlung der Hämophilie A erfolgt in erster Linie durch Ersatz des fehlenden Gerinnungsfaktors durch Faktorenkonzentrate z.B. durch Infusion von Faktor VIII, Faktor VIII-Komplexes oder vWF.

Das vWF-Syndrom besitzt mehrere Krankheitsbilder, die auf eine Unter- oder Überproduktion des vWFs zurückzuführen sind. So führt z.B. eine Überproduktion von vWF zur vermehrten Neigung von Thrombosen, wohingegen eine Unterversorgung durch die Abwesenheit oder eine Reduktion von hochmolekularen Formen des vWF begründet ist, die sich dadurch manifestiert, daß eine vermehrte Blutungsneigung und eine verlängerte Blutungszeit durch eine inhibierte Plättchenaggregation auftritt. Der Mangel an vWF kann auch eine phänotypische Hämophilie A hervorrufen, da der vWF ein wesentlicher Bestandteil des funktionellen Faktor VIII ist. In diesen Fällen ist die Halbwertszeit des Faktor VIII derart verringert, daß seine Funktion in der Blutgerinnungskaskade beeinträchtigt ist. Patienten mit von Willebrand-Syndrom (vWD) weisen daher oftmals Faktor VIII-Defizienz auf. Bei diesen Patienten ist die reduzierte Faktor VIII-Aktivität nicht die Konsequenz eines Defekts des X-chromosomalen Genes, sondern ist eine indirekte Folge der quantitativen und qualitativen Veränderung des vWF im Plasma. Die Unterscheidung zwischen Hämophile A und vWD kann normalerweise durch Messen des vWF-Antigens oder durch Bestimmen der Ristocetin-Kofaktor-Aktivität erfolgen. Sowohl der vWF-Antigengehalt als auch die Ristocetin-Kofaktor-Aktivität ist bei den meisten vWD-Patienten erniedrigt, wogegen sie in Hämophilie A-Patienten normal ist.

Konventionelle Methoden zur Therapie des von Willebrand-Syndroms erfolgen mit aus Plasma gewonnenem vWF, wobei es eine Reihe von Ansätzen gibt, vWD-Patienten mit gereinigtem vWF oder Faktor VIII/vWF-Komplex zu therapieren.

Die Reinigung von Faktor VIII oder Faktor VIII-Komplex aus Plasma oder Kryopräzipitat wird insbesondere dadurch erschwert, daß Faktor VIII nur in sehr geringen Mengen im Plasma vorkommt, extrem labil ist und die Assoziation von Faktor VIII mit vWF unter spezifischen Bedingungen reversibel ist. Faktor VIII wird durch Reinigung und Konzentrierung aus Plasma gewonnen, jedoch kann es abhängig vom Reinigungsverfahren zur Instabilität und Verlust der Faktor VIIl-Aktivität aufgrund der Trennung von vWF und Faktor VIII während der Reinigung kommen. Das Endprodukt ist daher oft eine Mischung aus stabilem Faktor VIII-Komplex und instabilem Faktor VIII, sowie von kontamierenden Proteinen, wie z.B. Fibrinogen, Fibronektin oder Vitamin K-abhängigen Proteinen, die durch die Reinigung nicht entfernt werden konnten. Aufgrund der Instabilität des gereinigten Komplexes werden Stabilisatoren, wie Albumin oder Aminosäuren etc., zugesetzt. Die Anwesenheit von kontaminierenden Proteinen und/oder Stabilisatoren im gereinigten Produkt reduzierten jedoch die spezifische Aktivität des Faktor VIII-Komplexes.

Die EP 0 468 181 beschreibt ein Verfahren zur Reinigung von Fakor VIII aus humanem Plasma durch Ionenaustauschchromatographie, Elution des Faktor VIII mit hoher lonenstärke bei saurem pH und Sammeln des Eluats in Anwesenheit eines Stabilisators, wie Heparin, Albumin und PEG und Lysin oder Histidin als Antiprotease. Die spezifische Aktivität nach Zugabe von Albumin sinkt jedoch von 300 bis 1200 U/mg Protein auf 18-24 U/mg Protein.

Madaras et al. (Haemostasis 7:321-331 (1978)) beschreiben ein Verfahren zur Reinigung von Faktor VIII an Heparin-Sepharose und Elution mit steigenden NaCl-Konzentrationen. Der so erhaltene Faktor VIII hatte jedoch nur geringe Aktivität.

Die US 5,252,709 beschreibt Verfahren zur Trennung von Faktor VIII, vWF, Fibronektin und Fibrinogen aus humanem Plasma, wobei zuerst Faktor VIII, vWF und Fibronektin an einen Ionentauscher von DEAE-Typ gebunden werden und anschließend durch steigende Salzkonzentrationen vom Ionenaustauscher getrennt eluiert werden.

Zimmerman et al. (US 4,361,509) beschreiben ein Verfahren zur Reinigung von Faktor VIII, wobei der Faktor VIII/vWF-Komplex an einen monoklonalen anti-vWF-Antikörper gebunden wird und Faktor VIII vom Komplex durch CaCI₂-Ionen dissoziiert wird. Der so erhaltene Faktor VIII wird anschließend über einen weiteren Chromatographieschritt in reiner Form gewonnen, muß jedoch durch Zugabe von humanem Albumin stabilisiert werden.

Durch die Expression von Faktor VIII in rekombinanten Zellen (Wood et al. (1984), Nature 312:330-337) konnte Faktor VIII gentechnisch hergestellt werden, jedoch konnte erst durch Zugabe oder Koexpression von vWF eine kommerziell einsetzbare Ausbeute an rekombinantem Faktor VIII erzielt werden. Zur Herstellung eines pharmazeutischen Präparates wird vWF jedoch wahrend des Reinigungsprozesses bis auf eine unwesentliche Restmenge vom Faktor VIII abgetrennt und der gereinigte rekombinante Faktor VIII mit Albumin stabilisiert (Griffith et al. (1991), Ann. Hematol 63:166-171).

Für den Einsatz zur Therapie von Patienten mit Hämophilie A, aber auch mit von Willebrand-Syndrom ist ein gereinigter Faktor VIII, komplexiert mit vWF, wünschenswert (Berntorp (1994), Haemostasis 24:289-297). Insbesondere wird immer wieder betont, daß in Präparaten ohne oder nur einem geringen Gehalt an vWF, eine verlängerte Blutungszeit und eine geringe Faktor VIII:C-Halbwertszeit in vivo zu beobachten ist. Eine Normalisierung von vWF in vivo ist wichtig, um die Konzentration von Faktor VIII im Plasma sowohl durch Reduktion der Faktor VIII-Eliminierungsrate als auch durch die Unterstützung der Freisetzung von endogenem Faktor VIII, aufrecht zu erhalten (Lethagen et al. (1992), Ann. Hematol. 65:253-259).

Die DE 3 504 385 beschreibt die Durchführung einer lonenaustauschchromatographie zur Reinigung von Faktor VIII/vWF-Komplex, wobei der Faktor VIII-Komplex über Sulfatgruppen gebunden und mit Citratpuffer, Calciumchlorid und NaCl-Gradient eluiert wird. Der Faktor VIII-Komplex wird dabei mit einer Konzentration von 0,5 M NaCl vom Träger eluiert.

Die EP 0 416 983 beschreibt die Gewinnung von Faktor VIII/vWF-Komplex aus menschlichem Plasma durch Ausfällung mit einer Kombination aus Bariumchlorid und Aluminumhydroxid und anschließende Anionenaustauschchromatographie an DEAE-Fraktogel.

In der EP 0 411 810 erfolgt die Reinigung von Faktor VIII/vWF-Komplex aus Kryopräzipitat mittels Heparin-Affinitätschromatographie und anschließende Elution des Komplexes mit Calciumchlorid. Eine Weiterentwicklung dieses Verfahrens wird in der WO 93/22337 beschrieben. Zur Entfernung von kontaminierenden Proteinen, wie Fibrinogen und Fibronektin, wird im Anschluß an die Elution mit CaCl₂ eine Glycin/NaCl-Fällung durchgeführt.

Zur Reinigung des Faktor VIII/vWF-Komplexes wurde ebenfalls vorgeschlagen, kontaminierende Proteine, wie etwa Fibrinogen, mit hohen Konzentrationen an Aminosäuren, insbesondere von Glycin, auszufällen, den in Lösung verbleibenden Faktor VIII/vWF-Komplex durch Zugabe eines calcium- und aminosäurehaltigen Puffers zu dissoziieren und anschließend Faktor VIII und vWF durch Anionenaustauschchromatographie getrennt voneinander zu gewinnen (WO 82/04395).

Die US 5,356,878 beschreibt die Herstellung von Faktor VIII-Komplex, bei dem kontaminierende Proteine (Fibrinogen, Vitamin-K-abhängige Faktoren oder Fibronektin) durch Fällung mit Al(OH)₃ und PEG abgetrennt, Faktor VIII-Komplex in Gegenwart von Glycin und NaCl chemisch virusinaktiviert und anschließend die nicht Faktor VIII-Komplex-spezifischen Proteine durch Gelfiltration entfernt werden.

Hornsey et al. (Thromb. Haemost. 57:102-105 (1987)) reinigten Faktor VIII/vWF mittels Immunaffinitätschromatographie und erreichten eine spezifische Aktivität von 45 U Faktor VIII/mg Protein und 60 U Ristocetin-Aktivität/mg Protein. Allerdings ist das Endprodukt mit 4% Fibrinogen und 2% Fibronektin und vom Träger abgelösten Maus-Antikörpern verunreinigt.

Mejan et al. (Thromb. Haemost. 59: 364-371 (1988)) schlugen vor, Faktor VIII/vWF-Komplex mittels Immunaffinität direkt aus Plasma zu reinigen. Der gereinigte Komplex wurde mit humanem Serumalbumin stabilisiert und anschließend lyophilisert. Mit den beschriebenen Elutionsbedingungen wurde jedoch eine teilweise Freisetzung der Antikörper von der Säule beobachtet, was zu einer Kontamination des Eluats mit monoklonalen Antikörpern führte und einen zweiten Reinigungsschritt zur Entfernung der Antikörper erforderte. Mejan et al. erreichten mit ihrem Verfahren eine etwa 1400-fache Anreicherung des Faktor VIII/vWF-Komplexes mit einer spezifischen Faktor VIII:C-Aktivität und Ristocetin-Aktivität von je 20 U/mg Protein, wobei das Produkt alle vWF-Multimere enthielt. Nach Stabilisierung des Komplexes mit 10 mg/ml Albumin wurde ein Stabilität von 3-4 Monaten bei -20°C beobachtet. Es wird jedoch immer wieder betont, daß eine besondere Schwierigkeit bei der Reinigung des Komplexes darin besteht, die Assoziation der Proteine zu erhalten, da beide Komponenten im Komplex instabil sind.

Harrison et al. (Thromb. Res. 50:295-304 (1988)) beschreibt die Reinigung von Faktor VIII/vWF-Komplex durch Chromatographie an Dextransulfat-Agarose.

Die EP 0 600 480 beschreibt die Reinigung von Faktor VIII/vWF-Komplex mittels Anionenaustauschchromatographie, wobei das Eluat, enthaltend Faktor VIII/vWF-Komplex, mit Heparin und Albumin stabilisiert und gegebenenfalls Lysin und Histidin als Antiproteasen zugegeben werden.

Kommerziell erhältliche Faktor VIII/vWF-Präparate weisen zum Teil keinen oder nur einen geringen Anteil an hochmolekularen vWF-Multimeren (vWF/HMW) auf und zeigen insbesondere in Abhängigkeit der Infusionszeit in vivo eine Reduktion der hochmolekularen vWF-Multimeren (Lethagen et al. (1992), Ann. Hematol. 65:253-259).

Die im Stand der Technik beschriebenen Faktor VIII-Präparate enthalten zwar zum größten Teil das gesamte vWF-Multimerpattern, jedoch variieren sie im Anteil an HMW-vWF und LMW-vWF und weisen sog. Triplett-Strukturen auf, die auf einen proteolytischen Abbau von vWF-Multimeren, insbesondere von vWF/HMW, hinweisen (Scott et al. (1993), Sem. Thromb. Hemost. 19:37-47, Baillod et al. (1992), Thromb. Res. 66:745-755, Mannucci et al. (1992), Blood 79:3130-3137). Die Stabilität dieser Präparate ist dadurch begrenzt.

Um die Präparate zu stabilisieren, entweder vor der Virusinaktivierung oder um ein lagerstabiles Präparat zu erhalten, wird immer wieder betont, daß die Zugabe eines Stabilisators, wie etwa Albumin, erforderlich ist.

Alle Faktor VIII-Konzentrate, die durch Reinigung des Proteins aus humanem Plasma erhalten werden, oder in Kontakt mit biologischem Material aus Säugetieren getreten sind, sind außerdem potentiell mit dem Risiko behaftet, mikrobiologische oder molekulare Pathogene, wie z.B. Viren, zu enthalten. Zur Herstellung eines sicheren Präparates ist daher auch immer eine Inaktivierung von pathogenen Organismen notwendig. Effektive Inaktivierungsverfahren können jedoch leicht auch zu einem Verlust der biologischen Aktivität des Faktor VIII-Komplexes führen. So stellten Palmer et al. fest (Thromb. Haemost. 63:392-402 (1990)), daß bei einer Hitzebehandlung zur effektiven Virusinaktivierung auch in Gegenwart eines Stabilisators mit einem Aktivitätsverlust zwischen 17% und 30% zu rechnen ist.

Es wird immer wieder betont, daß Faktor VIII/vWF-Konzentrate mit einer intakten Multimerstruktur möglicherweise einen guten Einfluß auf die Blutungszeit hätten, da sie die primäre Funktion des vWF, die Plättchenagglutination, ausführen und eine höhere Affinität zu den Plättchenrezeptoren Glykoprotein Ib und IIb/IIIa haben als niedermolekulare vWF-Multimere (LMW-vWF) (Mannucci et al. (1987), Americ. J. Hematology 25:55-65). Es besteht jedoch die Schwierigkeit, daß während des Herstellungsprozesses von Faktor VIII-Konzentraten ein Abbau insbesondere der HMW-vWF-Moleküle erfolgt.

Es besteht daher ein Bedarf an einem Faktor VIII-Komplex mit einer ausreichenden spezifischen Aktivität an Faktor VIII:C und vWF-Aktivität, der eine verbesserte Stabilität aufweist und der auch ohne Zugabe eines nicht-Faktor VIII/vWF-Komplex-spezifischen Stabilisators über einen längeren Zeitraum stabil bleibt.

Ziel der vorliegenden Erfindung ist es daher, einen Faktor VIII/vWF-Komplex mit einer verbesserten Stabilität zur Verfügung zu stellen.

Die Aufgabe wird erfindungsgemäß durch zur Verfügungstellen des Gegenstandes der Ansprüche, insbesondere den Ansprüche 1 bis 8, gelöst.

Die spezifische Plättchenagglutination gibt das Verhältnis von Ristocetin Co-Faktor-Aktivität und vWF-Antigengehalt wieder. Eine hohe spezifische Plättchenagglutinationsaktivität gibt daher die spezifische Aktivität der Multimeren an. Im Rahmen der vorliegenden Erfindung konnte sowohl für plasmatischen vWF (p-vWF) als auch rekombinanten vWF (r-vWF) bzw. Faktor VIII/vWF-Komplex gezeigt werden, daß bei einem hohen Multimerisierungsgrad des vWF die spezifische Plättchenagglutination (RistCoF / vWF:Ag) wesentlich erhöht ist im Vergleich zu niedermolekularen Multimeren.

Niedermolekularer p-vWF (p-vWF/LMW) und niedermolekularer r-vWF (r-vWF/LMW) besitzen nur sehr geringe Plättchenagglutination.

Diese Situation kann dadurch verdeutlicht werden, daß durch die sehr kurze vWF-Kette es zu keiner stabilen Verbindung mehrerer Plättchen kommt. Demgegenüber können hochmolekulare (lange) vWF-Multimere mehrere Plättchen miteinander stabil verbinden.

Es wurde gezeigt, daß sowohl hochmolekularer p-vWF (p-vWF/HMW) als auch hochmolekularer r-vWF (r-vWF/HMW) in konzentrationsabhängiger Weise an Plättchen binden und eine höhere spezifische Plättchenagglutinationsaktivität aufweisen als niedermolekulare vWF-Multimere.

Der erfindungsgemäße stabile Faktor VIII/vWF-Komplex besitzt daher eine spezifische vWF-Plättchenagglutinationsaktivität von mindestens 50 U/mg vWF:Ag.

Im Plasma kommt Faktor VIII/vWF im Komplex in einem molaren Verhältnis von ungefähr 1:50 vor. Es wurde festgestellt, daß dieses Verhältnis im Plasma notwendig ist, um einen guten Schutz gegen proteolytischen Abbau, insbesondere durch Protein C, zu gewähren (Vlot et al. (1995), Blood 85:3150-3157).

Gemäß einem weiteren Aspekt der vorliegenden Erfindung besitzt der erfindungsgemäße Faktor VIII/vWF-Komplex ein molares Verhältnis von Faktor VIII zu vWF zwischen 0,01 und 100. Dies bedeutet, daß der Komplex ein Verhältnis von Faktor VIII-Molekülen zu vWF-Molekülen zwischen 1:100 bzw. 100:1 aufweist. Vorzugsweise liegt das molare Verhältnis von Faktor VIII zu vWF zwischen 1:30 und 1:70 beträgt, besonders bevorzugt bei 1:50, wobei durch den hohen Anteil an vWF/HMW im Komplex ein optimales Verhältnis zum Schutz gegen proteolytischen Abbau erhalten wird.

Erfindungsgemäß wird weiter ein stabiler Faktor VIII/vWF-Komplex zur Verfügung gestellt, der hochmolekulare plasmatische vWF-Multimere mit einer Duplett-Struktur enthält.

Es wurde im Rahmen der vorliegenden Erfindung festgestellt, daß aus Plasma oder Kryopräzipitat ein durch ein chromatographisches Verfahren gereinigter Faktor VIII-Komplex erhalten wird, der aus vWF-Multimermolekülen besteht, die eine Duplettstruktur aufweisen. Dies war insbesondere deshalb überraschend, da aus Plasma oder Kryopräzipitat gereinigter vWF oder Faktor VIII/vWF-Komplex nur mit einer Triplettstruktur der vWF-Multimeren bekannt war. Diese Triplettstrukturen entstehen durch proteolytischen Abbau von vWF-Multimeren und weisen auf eine Instabilität der vWF-Multimere hin. Von Palmer et al. (Thromb. Haemost. 63:392-402 (1990)) wurde beschrieben, daß bei der Herstellung von Faktor VIII-Konzentrat aus heparinisiertem Plasma und anschließender Virusinaktivierung das normale Triplettmuster sich verändert, und die Intensität der Triplett-Bande mit dem niedrigsten Molekulargewicht stark zunimmt, was auf einen verstärkten proteolytischen Abbau der vWF-Multimere hinweist. Im Gegensatz dazu wird bei der vorliegenden Erfindung ein Faktor VIII/vWF zur Verfügung gestellt, dem dieses vWF-Abbauprodukt vollkommen fehlt und der im wesentlichen die 2 Banden des ursprünglichen Tripletts mit dem höheren Molekulargewicht enthält.

Gemäß einer weiteren Ausführungsform der Erfindung wird ein stabiler Faktor VIII/vWF-Komplex zur Verfügung gestellt, der hochmolekulare rekombinante vWF-Multimere mit einer Singulett-Struktur enthält. Es wurde durch Multimeranalyse festgestellt, daß die vWF-Multimeren von rekombinantem vWF lediglich eine Singulettbande aufweisen, eine hohe strukturelle Integrität besitzen und frei sind von jeglichen proteolytischen vWF-Abbauprodukten.

Der erfindungsgemäße stabile Faktor VIII/vWF-Komplex ist vorzugsweise frei von Plasmaproteinen, insbesondere von Plasmaproteasen, und frei von Fibrinogen und Fibronektin. Da Plasmaproteasen und Plasmaproteine, insbesondere aktivierte Plasmaproteine, wie Protein C, Faktor lla oder Faktor IXa, vWF oder Faktor VIII proteolytisch degradieren und der erfindungsgemäße Komplex frei von Plasmaproteinen ist, besitzt er eine erhöhte Stabilität und Integrität der Proteine im Komplex.

Der erfindungsgemäße Komplex besitzt gegen proteolytischen Abbau eine erhöhte Resistenz, wodurch er beispielsweise bei Raumtemperatur, für mindestens 48 Stunden, vorzugsweise für mindestens 6 Tage stabil ist, und in lyophilisierter Form bei 4°C oder Raumtemperatur mehr als 2 Jahre lagerstabil ist.

Der erfindungsgemäße Faktor VIII/vWF-Komplex ist derart stabil, daß er als virussicherer Komplex zur Verfügung gestellt werden kann. Die Virussicherheit ist durch Verfahrensschritte zur Behandlung des Komplexes zur Inaktivierung von Viren bzw. Abreicherung von Viren gewährleistet.

Zur Inaktivierung von Viren eignet sich insbesondere eine Hitzebehandlung in Lösung bzw. festem, vorzugsweise lyophilisiertem Zustand, welche sowohl lipidumhüllte als auch nicht-lipidumhüllte Viren verläßlich inaktivieren kann. Beispielsweise wird der erfindungsgemäße Komplex gemäß der EP 0 159 311 in festem, nassen Zustand hitzebehandelt. Andere Verfahren zur Virusinaktivierung umfassen auch die Behandlung mit Detergenzien oder chaotropen Substanzen, beispielsweise gemäß der EP 0 519 901, WO 94/13329, DE 44 34 538 und EP 0 131 740.

Die Erfindung betrifft also ein stabiles, virussicheres Faktor VIII-Komplex-Konzentrat, enthaltend insbesondere hochmolekulare vWF-Multimere mit hoher struktureller Integrität. Die hochmolekularen vWF-Multimeren bestehen vorzugsweise aus einer Singulett- oder einer Duplett-Struktur und sind frei von proteolytischen Abbauprodukten des vWF. Es hat sich überraschenderweise gezeigt, daß das erfindungsgemäße Faktor VIII-Komplex-Konzentrat derart stabil ist, daß eine Behandlung zur Virusinaktivierung, wie etwa oben beschrieben, die Stabilität der Proteine, insbesondere der hochmolekularen vWF-Multimere im Komplex nur unwesentlich beeinträchtigt und daß dadurch die spezifische Aktivität von Faktor VIII:C und vWF-Ristocetin-Aktivität im Faktor VIII-Komplex oder Faktor VIII-/Komplex-Konzentrat bei einem Virusinaktivierungsschritt nur bis zu maximal 10% herabgesetzt ist. Bei bisher bekannten Faktor VIII-Komplex-Konzentraten war bei der Virusinaktivierung mit einem Verlust der Aktivität zwischen 20 und 30 % zu rechnen. Der erfindungsgemäße Faktor VIII/vWF-Komplex zeigte im Neoantigentest keine Veränderungen der Antigenstruktur nach dem Virusinaktivierungsschritt, was die Stabilität der Proteine im Komplex bestätigt. Aufgrund der hohen Stabilität der hochmolekularen vWF-Multimeren ist auch eine hohe spezifische Plättchenagglutinationsaktivität von mindestens 50 U/mg vWF:Ag im erfindungsgemäßen Faktor VIII-Komplex-Konzentrat gewährleistet.

Gemäß einer besonderen Ausführungsform enthält das erfindungsgemäße stabile, virussichere Faktor VIII-Komplex-Konzentrat Faktor VIII und vwF in einem molaren Verhältnis von Faktor VIII zu vWF zwischen 0,01 und 100, vorzugsweise zwischen 0,05 und 1. Das Faktor VIII-Komplex-Konzentrat ist insbesondere frei von Plasma-proteinen, insbesondere von Plasmaproteasen, und frei von mikrobiologischen und molekularbiologischen Pathogenen.

Zur Verbesserung der Stabilität von gereinigten Proteinen werden gewöhnlicherweise Stabilisatoren, wie etwa Albumin, zugesetzt. Dabei wird jedoch durch die Zugabe des Fremdproteins die spezifische Aktivität des gereinigten Proteins herabgesetzt. vWF ist ein natürlicher Bestandteil des Faktor VIII-Komplexes. Es wurde gefunden, daß durch Zugabe von hochmolekularen vWF-Multimeren (vWF/HMW) zu einer gereinigten Faktor VIII-Fraktion aus Plasma oder aus rekombinanten Zellkulturen die Stabilität von Faktor VIII erhöht wird. Ebenso wurde gefunden, daß durch Zugabe von vWF/HMW zu einem gereinigten Faktor VIII-Komplex die Stabilität des Komplexes verbessert werden kann. Auf den Zusatz von üblicherweise verwendeten Stabilisatoren kann daher verzichtet werden. In Ausnahmefällen kann gegebenenfalls auch während der Gewinnung ein Proteaseinhibitor zugesetzt werden, um die intakte Struktur, insbesondere der vWF/HMW zu erhalten.

Erfindungsgemäß wird weiter eine pharmazeutische Zusammensetzung, enthaltend einen erfindungsgemäßen stabilen Faktor VIII/vWF-Komplex oder ein erfindungsgemäßes virussicheres, stabiles Faktor VIII-Komplex-Konzentrat, zur Verfügung gestellt.

In einer besonderen Ausführungsform enthält die pharmazeutische Zusammensetzung einen physiologisch akzeptablen Träger oder Puffer. Die Formulierung der erfindungsgemäßen pharmazeutischen Präparation kann in an sich bekannter und üblicher Weise erfolgen, z.B. mit Hilfe von Salzen und gegebenenfalls Aminosäuren, aber auch in Gegenwart von Tensiden vorgenommen werden. Aufgrund der oben beschriebenen hohen Stabilität des Komplexes kann auf die üblicherweise verwendeten Stabilisatoren oder Protease-Inhibitoren in der pharmazeutischen Zusammensetzung gegebenenfalls auch verzichtet werden.

Der erfindungsgemäße stabile Faktor VIII/vWF-Komplex wird vorzugsweise als hochreines Produkt erhalten, welches durch chromatographische Reinigungsverfahren erhalten wird. Die chromatographische Reinigung erfolgt insbesondere durch lonenaustauschchromatographie und/oder Affinitätschromatographie. Dafür können u.a. Materialien zum Anionenaustausch, wie synthetische Trägermaterialien oder Träger auf Kohlehydratbasis, mit Liganden, wie DEAE, TMAE-, QAE-, Q-, oder Aminoalkylgruppen herangezogen werden, bzw. für die Affinitätschromatographie Träger mit immobilisierten Substanzen, die eine spezifische Affinität für vWF aufweisen. Geeignete Affinitätsmaterialien enthalten beispielsweise Heparin.

Gemäß einem weiteren Aspekt der vorliegenden Erfindung wird daher ein Verfahren zur Gewinnung von stabilem Faktor VIII/vWF-Komplex zur Verfügung gestellt. Dabei wird Faktor VIII/vWF-Komplex aus einer Proteinlösung bei einer niedrigen Salzkonzentration an einen Affinitätsträger, vorzugsweise einen Heparin-Affinitätsträger, gebunden und stabiler Faktor VIII/vWF-Komplex bei einer hohen Salzkonzentration gewonnen. Der Komplex wird dabei vorzugsweise an immobilisiertes Heparin bei einer Salzkonzentration von ≤ 150 mM gebunden und bei einer Salzkonzentration von ≥ 200 mM und ≤ 300 mM eluiert. In einer besonders bevorzugten Ausführungsform der vorliegenden Erfindung wird Faktor VIII/vWF-Komplex in einem CaCl₂-freien Puffersystem durchgeführt. Auf diese Weise lassen sich Faktor VIII/vWF-Komplexe, enthaltend niedermolekulare vWF-Moleküle bzw. hochmolekulare vWF-Moleküle, selektiv voneinander trennen und Faktor VIII/vWF-Komplex, enthaltend insbesondere hochmolekulare vWF-Moleküle, kann bei einer höheren Salzkonzentration gewonnen werden. Für die Elution sind lösliche ein- und zweiwertige Salze verwendbar. Vorzugsweise wird NaCl verwendet. Calciumsalze sind für die Elution nicht geeignet.

Das erfindungsgemäße Verfahren wird vorzugsweise an einer Heparin-Affinitätschromatographie-Säule durchgeführt. Für die Affinitätschromatographie kann jeder Träger, an den Heparin gebunden werden kann, verwendet werden. Als gut geeignet erwiesen sich zum Beispiel AF-Heparin Toyopearl® (ein synthetisches, großporiges, hydrophiles Polymer auf der Basis von Methacrylat) (Tosohaas), Heparin EMD Fraktogel® (ein synthetisches, hydrophiles Polymer auf der Basis von Ethylenglykol, Methacrylat und Dimethylacrylat) (Merck) oder Heparin Sepharose Fast Flow® (enthaltend natürliche Dextran-bzw. Agarosederivate) (Pharmacia).

Im erfindungsgemäßen Verfahren wird als Puffersystem eine Pufferlösung bestehend aus Puffersubstanzen, insbesondere Tris/HCl, Phosphatpuffer oder Citratpuffer, und gegebenenfalls Salz verwendet, die vorzugsweise frei von Stabilisatoren, Aminosäuren oder anderen Zusätzen ist.

Die Affinitätschromatographie wird vorzugsweise in einem pH-Bereich von 6,0 bis 8,5, vorzugsweise bei einem pH-Wert von 7,4 durchgeführt.

Beim erfindungsgemäßen Verfahren wird eine Proteinlösung, enthaltend Faktor VIII/vWF-Komplex, wie etwa eine Plasmafraktion, ein Kryopräzipitat oder ein zellfreier Kulturüberstand von transformierten Zellen, eingesetzt. Die Lösung kann auch eine angereicherte Proteinfraktion eines chromatographischen Verfahrens sein.

Nach dem erfindungsgemäßen Verfahren zur Gewinnung des Faktor VIII/vWF-Komplexes läßt sich auf effiziente und einfache Weise ein Faktor VIII/vWF-Komplex, im wesentlichen enthaltend hochmolekulare vWF-Multimere, erhalten. Nach diesem Verfahren läßt sich deshalb ein physiologisch besonders aktiver Faktor VIII/vWF-Komplex mit guten Ausbeuten und in hoher Reinheit herstellen. Der so gewonnene Faktor VIII/vWF-Komplex zeichnet sich insbesondere durch eine spezifische Aktivität von Faktor VIII:C von mindestens 50 U/mg Faktor VIII:Ag und eine spezifische vWF-Plättchenagglutinationsaktivität von mindestens 50 U/mg vWF aus und ist insbesondere frei von niedermolekularen vWF-Multimeren und vWF-Abbauprodukten.

Gemäß einem weiteren Aspekt der vorliegenden Erfindung wird ein Verfahren zur Gewinnung von stabilem Faktor VIII/vWF-Komplex zur Verfügung gestellt, bei dem Faktor VIII/vWF-Komplex aus einer unreinen Proteinlösung an einen Anionenaustauscher gebunden und kontaminierende Plasmaproteine bei einer Salzkonzentration von ≤ 200 mM in Anwesenheit von Calciumsalz selektiv eluiert werden. Anschließend wird Faktor VIII/vWF-Komplex vom Anionenaustauscher mit einer Salzkonzentration zwischen ≤ 200 und ≤ 400 mM erhalten. Dabei wird ein Faktor VIII/vWF-Komplex, im wesentlichen enthaltend hochmolekulare vWF-Multimere, gewonnen.

Zur Durchführung des erfindungsgemäßen Verfahrens kann als unreine Proteinlösung, enthaltend Faktor VIII/vWF-Komplex, z.B. eine Plasmafraktion, ein Kryopräzipitat oder ein zellfreier Kulturüberstand von transformierten Zellen eingesetzt werden.

Die durch die Calciumsalze entfernten kontaminierenden Proteine sind insbesondere Plasmaproteine, darunter Vitamin K-abhängige Faktoren, wie z.B. Faktor II, Faktor IX, Protein C, Protein S, Plasmaproteasen wie Plasminogen, Fibronektin oder Fibrinogen. Das Entfernen der unspezifischen Proteine erfolgt insbesondere mit CaCl₂ als Calciumsalz im Elutionsmittel in einer Konzentration zwischen 1 mM und 15 mM, vorzugsweise von 10 mM.

Im Rahmen der vorliegenden Erfindung wurde gefunden, daß das bisher eingesetzte Verfahren der Aluminiumhydroxid-Behandlung zur Abtrennung von Vitamin K-abhängigen Proteinen, Fibrinogen oder Fibronektin nicht ausreichend ist, um diese Proteine vollständig zu entfernen. Durch die Elution in Anwesenheit von Calciumchlorid wurde jedoch gewährleistet, daß diese Plasmaproteine im wesentlichen eliminiert werden und ein Plasmaprotein-freier Faktor VIII/vWF-Komplex gewonnen wird.

Die Anionenaustauschchromatographie wird vorzugsweise in einem pH-Bereich von 6,0 bis 8,5, vorzugsweise bei einem pH-Wert von 7,4, vorgenommen.

Die Elution des bei der Anionenaustauschchromatographie an den Anionenaustauscher gebundenen Faktor VIII/vWF-Komplexes erfolgt vorzugsweise durch Erhöhung der Salzkonzentration.

Als Anionenaustauscher wird vorzugsweise ein Anionenaustauscher vom quaternären Aminotyp, insbesondere ein Fraktogel mit Tentkelstruktur und insbesondere EMD-TMAE-Fraktogel verwendet.

Vorzugsweise wird Faktor VIII/vWF-Komplex an den Anionenaustauscher bei einer Salzkonzentration von ≤ 200 mM gebunden und bei einer Salzkonzentration von ≥ 270 mM, vorzugsweise bei ≥ 350 mM, Faktor VIII/vWF-Komplex, im wesentlichen enthaltend vWF/HMW, eluiert. Als Salze sind lösliche ein- und zweiwertige Salze einsetzbar, wobei NaCl bevorzugt ist.

Vorzugsweise wird der durch die Anionenaustauschchromatographie gereinigte Faktor VIII-Komplex weiterhin durch eine Affinitätschromatographie, vorzugsweise an immobilisertem Heparin, in einer Pufferlösung, bestehend aus Puffersubstanzen und gegebenenfalls Salz, chromatographisch gereinigt.

In einer besonderen Ausführungsform des erfindungsgemäßen Verfahrens wird zuerst eine aus Kryopräzipitat gewonnene Faktor VIII/vWF-Komplex-haltige Fraktion an einen Anionenaustauscher gebunden und nach Abtrennen der Begleitproteine, insbesondere der Plasmaproteine, in angereicherter Form Faktor VIII/vWF-Komplex, enthaltend im wesentlichen hochmolekulare vWF-Multimere, eluiert. In einem weiteren Reinigungsschritt wird das Faktor VIII/vWF-Komplex-haltige Eluat mit einem Affinitätsträger mit kovalent gebundenem Heparin in Kontakt gebracht, wobei der Komplex an den Träger bindet. Nach Entfernen von Fremdsubstanzen und Fremdproteinen durch ein geeignetes Elutionsmittel wird der Faktor VIII-Komplex vom Affinitätsträger mittels einem ein- oder zweiwertigen Salz, vorzugsweise NaCl, in einem Puffersystem eluiert.

In einer weiteren besonderen Ausführungsform erfolgt die Durchführung des Verfahrens mit aus rekombinanten Zellen gewonnenem Faktor VIII/vWF-Komplex. Dazu kann ein zellfreier Kulturüberstand von Zellen, die Faktor VIII und vWF koexprimieren, oder von kokultivierten Zellen, die mit Faktor VIII einerseits und mit vWF andererseits transformiert sind, verwendet werden.

Durch das erfindungsgemäße Verfahren zur Gewinnung eines hochreinen stabilen Faktor VIII-Komplexes läßt sich auf einfache und effiziente Weise ein hochreiner Faktor VIII/vWF-Komplex, der frei ist von Antikörpern, frei von Plasmaproteinen, physiologisch aktiv und frei von mikrobiologisch und molekularbiologischen Pathogenen ist, erhalten.

Der aus Plasma oder aus rekombinanten Zellen gewonnene Faktor VIII/vWF-Komplex enthält gemäß der vorliegenden Erfindung insbesondere hochmolekulare vWF-Multimere, und ist frei von vWF-Abbauprodukten. Wird der Faktor VIII/vWF-Komplex aus Plasma oder Kryopräzipitat gewonnen, so bestehen die vWF/HMW insbesondere aus Duplettstrukturen mit hoher Stabiliät. Aus rekombinanten Zellen gewonnener Faktor VIII/vWF-Komplex enthält hochmolekulare vWF-Moleküle mit einer Singulettstruktur, hoher Stabilität und struktureller Integrität.

Mittels definierter Chromatographieschritte ist es daher gemäß der vorliegenden Erfindung möglich, FVIII/vWF, frei von anderen Gerinnungsfaktoren und weiteren Plasma-Proteasen, zu gewinnen. Dies wirkt sich insbesondere auf die Reinheit und Stabilität des Präparates günstig aus. Der erfindungsgemäß erhaltene Faktor VIII/vWF-Komplex liegt daher in einer besonders reinen Form vor. Die Reinheit des Komplexes ist vorzugsweise mindestens 90%, besonders bevorzugt 95%.

Dadurch, daß der gewonnene Faktor VIII/vWF-Komplex durch den hohen Gehalt an hochmolekularen vWF-Multimeren und die Abwesenheit von vWF-Abbauprodukten, Fremdproteinen, wie z.B. Plasmaproteinen, besonders stabil ist, ist es nicht unbedingt erforderlich, Stabilisatoren zum gereinigten Produkt zuzugeben. Dadurch wird die spezifische Aktiviät des reinen Produkts z.B. bei der Formulierung der pharmazeutischen Zusammensetzung, nicht herabgesetzt und es wird vermieden, daß durch die Zugabe von Fremdproteinen gegebenenfalls Verunreinigungen oder infektiöse Partikel in das Produkt eingebracht werden.

In einem weiteren Aspekt der Erfindung wird ein Verfahren zur Herstellung eines stabilen Faktor VIII/vWF-Komplexes zur Verfügung gestellt. Dabei wird einem über ein chromatographisches Verfahren gereinigten Faktor VIII oder Faktor VIII-Komplex eine gereinigte hochmolekulare Fraktion von vWF-Molekülen zugesetzt, wodurch ein Faktor VIII/vWF-Komplex mit einem molaren Verhältnis von Faktor VIII zu vWF/HMW zwischen 0,01 (entspricht 1 Faktor VIII:100 vWF) und 100 (entspricht 100 Faktor VIII:1 vWF), vorzugsweise zwischen 0,03 (1:30) und 0,07 (1:70), besonders bevorzugt von 0,05 (1:50) erhalten wird.

Der über ein chromatographisches Verfahren gereinigte Faktor VIII oder Faktor VIII-Komplex kann dabei aus einer Plasmafraktion, einem Kryopräzipitat oder aus einem zellfreien Zellkulturüberstand von transformierten Zellen stammen.

Die für das Verfahren zur Herstellung des stabilen Komplexes eingesetzte hochmolekulare Fraktion von vWF-Molekülen kann sowohl aus Plasma, einer Plasmafraktion, einem Kryopräzipitat oder einem zellfreien Kulturüberstand von transformierten Zellen stammen. Die gereinigte hochmolekulare Fraktion von vWF-Molekülen enthält vorzugsweise eine spezifische Plättchenagglutinationsaktivtät von mindestens 50 U/mg vWF:Ag, besonders bevorzugt von mindestens 80 U/mg vWF:Ag.

Zur Herstellung des stabilen Faktor VIII-Komplexes wird eine gereinigte Fraktion, enthaltend Faktor VIII oder Faktor VIII/vWF-Komplex, mit einer gereinigten hochmolekularen Fraktion von vWF-Molekülen in einem gewünschten molaren Verhältnis gemischt. Dazu wird in den jeweiligen Fraktionen der Gehalt an vWF, Faktor VIII, die Faktor VIl-Aktivität und spezifische vWF-Aktivität bestimmt und durch Zugabe der entsprechende Menge an vWF/HMW das gewünschte Mischungsverhältnis eingestellt. Vorzugsweise erfolgt die Mischung derart, daß ein Faktor VIII/vWF-Komplex entsteht, der ein molares Verhältnis von Faktor VIII zu vWF zwischen 1:100 und 100:1, vorzugsweise von 1:50 aufweist.

Gemäß der vorliegenden Erfindung kann jedoch auch ein Faktor VIII/vWF-Komplex hergestellt werden, der ein bestimmtes Verhältnis von spezifischer Faktor VIII:C-Aktivität zu spezifischer vWF-Plättchenagglutinationsaktivität aufweist. Besonders bevorzugt ist dabei ein Komplex, der ein Verhältnis der spezifischen Aktivitäten von 1:1 aufweist. Das gewünschte Mischungsverhältnis herzustellen, liegt dabei im allgemeinen Wissensstand eines Fachmannes.

Der gemäß der vorliegenden Erfindung zur Verfügung gestellte stabile virussichere Faktor VIII-Komplex sowie das Faktor VIII-Komplex-Konzentrat können sowohl für die Behandlung von Hämophilie A als auch zur Behandlung des von Willebrand-Syndroms eingesetzt werden. Da das erfindungsgemäße Faktor VIII-Komplex-Präparat im wesentlichen hochmolekulare vWF-Moleküle enthält, eignet es sich insbesondere für die Behandlung von vWD Typ II.

Aufgrund des hohen Anteils von hochmolekularen vWF-Multimeren besitzt der erfindungsgemäße Komplex auch eine sehr gute Pharmokinetik, da die vWF/HWM eine höhere spezifische Plättchenagglutination und Stabilität in vitro und in vivo besitzen und sowohl in vitro als auch in vivo Faktor VIII stabilisieren. Durch die Stabilität und strukturelle Integrität der vWF-Multimere im Komplex ist eine verbesserte Halbwertszeit des vWF und insbesondere auch von Faktor VIII gegeben, wodurch gegebenenfalls die Intervalle einer Verabreichung des erfindungsgemäßen pharmazeutischen Präparates reduziert werden können. Damit wird insbesondere das Auftreten von inhibitorischen Antikörpern gegen Faktor VIII bei Hämophilie A-Patienten, z.B. durch häufige Gaben von Faktor VIII-Konzentraten, verhindert.

Die Erfindung wird anhand der nachfolgenden Beispiele sowie der Zeichnungsfiguren näher erläutert, wobei sie jedoch nicht auf diese beschränkt ist.

Es zeigen:

**Figur 1**: SDS-PAGE-Analyse des vWF-Multimermusters der einzelnen Fraktionen vor und nach Anionenaustauschchromatographie.

**Figur 2**: Nachweis von Faktor II in einzelnen Fraktionen vor und nach Anionenaustauschchromatographie und nach Entfernen von Faktor II durch Calciumchlorid-Elution.

**Figur 3**: Nachweis von Protein S in einzelnen Fraktionen vor und nach Anionenaustauschchromatographie und nach Entfernen von Protein S durch Calciumchlorid-Elution.

**Figur 4**: Nachweis von Faktor IX in einzelnen Fraktionen vor und nach Anionenaustauschchromatographie und Entfernen von Faktor IX durch Calciumchlorid-Elution.

**Figur 5**: Nachweis von Plasminogen in einzelnen Fraktionen vor und nach Anionenaustauschchromatographie und Entfernen von Plasminogen durch vorangegangene Lysin-Sepharose.

**Figur 6**: SDS-PAGE-Analyse des vWF-Multimermusters der einzelnen Fraktionen vor und nach Heparin-Affinitätschromatographie.

**Figur 7**: Multimeranalyse von p-vWF und r-vWF vor und nach Heparin-Affinitätschromatographie.

**Figur 8**: Vergleich der Bindung von r-vWF/HMW und p-vWF/HMW an Plättchen und graphische Darstellung der zugegebenen Menge an vWF und Plättchen-gebundener Menge an vWF.

**Figur 9**: Bindung von p-vWF/HMW und r-vWF/HMW an Plättchen und Multimeranalyse.

### Beispiel 1:

### Reinigung von Faktor VIII/vWF-Komplex durch Anionenaustauschchromatographie

10 g Kryopräzipitat wurden mit 70 ml Na-Acetat, pH 7,0, 160 mM NaCl und 50 U/ml Heparin bei 30°C für 10 min gelöst und bis zum vollständigen Auslösen bei Raumtemperatur für weitere 30 min inkubiert. Die Lösung wurde auf 15°C abgekühlt und bis zur Entfernung ungelöster Bestandteile zentrifugiert und der Überstand mit Aluminiumhydroxidgel behandelt. Die Lösung wurde an einen Fractogel-EMD-TMAE-Anionenaustauscher gebunden und zur Entfernung von Fremdproteinen der Anionenaustauscher mit 180 mM NaCl und 200 mM NaCl gewaschen. vWF und FVIII wurden als Komplex anschließend mit 400 mM NaCl eluiert. Die Faktor VIII:C- und vWF: RistoCoF-Aktivität des Ausgangsmaterials und der einzelnen Fraktionen wurden bestimmt und sind in Tabelle 1 zusammengefaßt.

**Tabelle 1:**

| **Faktor VIII:C und vWF:RistoCoF-Aktivität des Aluminiumhydroxid-Überstandes und der Fraktionen der Anionenaustauschchromatographie** | | | |
|---|---|---|---|
| Probe | Volumen | vWF:RistCoF | FVIII:C |
| | (ml) | (mU/ml) | (mU/ml) |
| Alu-Überstand | 147 | 1060 | 2970 |
| 180 mM Eluat | 254 | - | - |
| 200 mM Eluat | 210 | - | - |
| 400 mM Eluat | 132 | 1590 | 2890 |

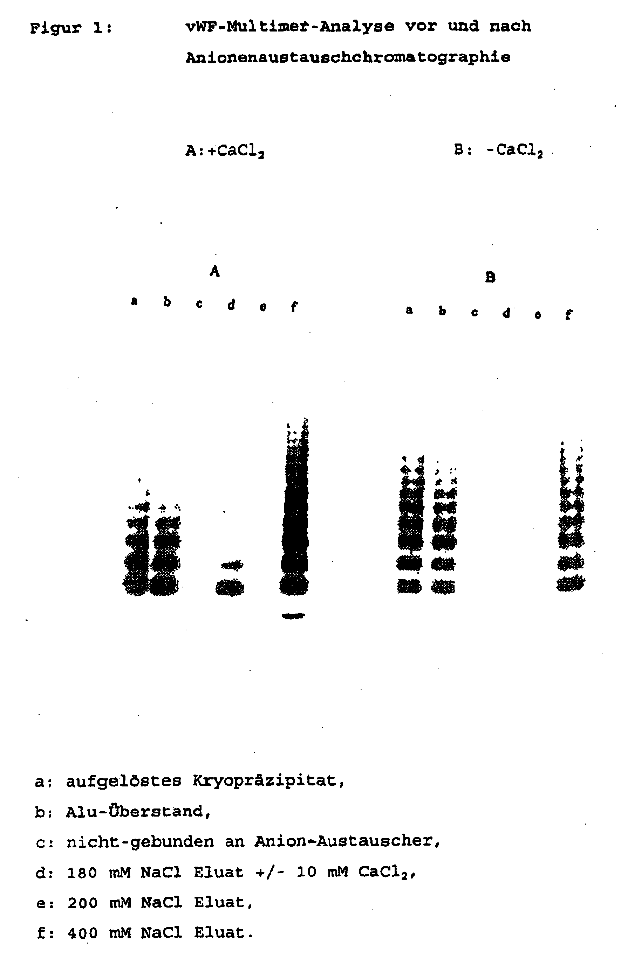

Durch die Anionenaustauschchromatographie konnte Faktor VIII/vWF-Komplex mit einer erhöhten vWF:RistoCoF-Aktivität erhalten werden. Zur Untersuchung des vWF-Polymermusters wurden die einzelnen Fraktionen der Anionenaustauschchromatographie über SDS-PAGE (Laemmli (1970), Nature 227:680-685) analysiert (Figur 1 B). Das Polymermuster des vWF im gereinigten Faktor VIII/vWF-Komplex zeigt ein identes Bandenmuster und damit die gleiche vWF-Polymerzusammensetzung wie im Kryopräzipitat. Die Reinigung führte daher nicht zu einem proteolytischen Abbau von hochmolekularen vWF-Multimeren im Komplex.

### Beispiel 2:

### Entfernen von Vitamin K-abhängigen Proteinen und Gewinnung von hochreinem Faktor VIII/vWF-Komplex

Ziel der Untersuchungen war es, einen FVIII/vWF-Komplex zu gewinnen, frei von Proteasen und anderen Gerinnungsfaktoren. Wie in Beispiel 1 beschrieben, wurde aufgelöstes Kryopräzipitat mit Aluminium-Hydroxid behandelt, und anschließend durch Anionen-Austauschchromatographie gereinigt. Zum Entfernen von nicht Faktor VIII/vWF-Komplex-spezifischen Proteinen wurden bei der Elution mit 180 mM NaCl 10 mM CaCl₂ zugesetzt. Die Faktor VIII:Cund vWF:RistoCoF-Aktivität des Ausgangsmaterials und der einzelnen Fraktionen wurden bestimmt und sind in Tabelle 2 zusammengefaßt.

**Tabelle 2:**

| **Faktor VIII:C- und vWF:RistoCoF-Aktivität des Ausgangsmaterials und der einzelnen Fraktionen der Anionenaustauschchromatographie** | | | |
|---|---|---|---|
| Probe | Volumen | vWF:RistCoF | FVIII:C |
| | (ml) | (mU/ml) | (mU/ml) |
| Alu-Überstand | 146 | 1590 | 4250 |
| 180 mM Eluat/ 10 mM CaCI₂ | 195 | - | - |
| 200 mM Eluat | 127 | - | - |
| 400 mM Eluat | 83 | 2120 | 5140 |

Die Eluate wurden mittels SDS-PAGE auf ihr vWF-Multimer-Muster untersucht (Figur 1A).

Aus der Multimer-Analyse geht hervor, daß das 400 mM-Eluat die hochmolekularen vWF-Multimere enthält, und ausgehend vom Kryopräzipitat kein Verlust, insbesondere an hochmolekularen vWF-Multimeren, auftritt. Durch den Zusatz von CaCl₂-Ionen wird niedermolekularer vWF abgetrennt und ein Faktor VIII-Komplex, enthaltend einen höheren Anteil an hochmolekularen vWF-Molekülen, erhalten (Fig. 1A). Damit ist gezeigt, daß durch das Reinigungsverfahren ein proteolytischer Abbau der hochmolekularen vWF-Multimere vermieden wird und durch Zugabe von CaCl₂-Ionen selektiv niedermolekulare vWF-Moleküle, wie etwa Dimere oder Tetramere, entfernt werden können, wodurch ein Faktor VIII-Komplex, im wesentlichen enthaltend hochmolekulare vWF-Multimere, gewonnen wird.

Die einzelnen Reinigungsstufen, enthaltend Faktor VIII/vWF-Komplex, wurden auf die Anwesenheit von Vitamin K-abhängigen Proteinen vor und während der Anionen-Austauschchromatographie analysiert. Dazu wurden einzelne Reinigungsstufen und die einzelnen Fraktionen über ein SDS-PAGE aufgetrennt, die Proteine auf eine Membran transferiert und mittels Westernblot-Analyse die Vitamin K-abhängigen Proteine detektiert.

### a. Nachweis von Faktor II in den einzelnen Reinigungsstufen

Zum Nachweis von Faktor II in den einzelnen Fraktionen wurde polyklonales Serum gegen Faktor II (Assera Faktor II, Stago) als 1. Antikörper eingesetzt und mit alkalischer Phosphatase konjugiertem polyklonalen Ziegen-anti-Kaninchen-IgG-HRP-Konjugat (Fa. Bio-Rad) als 2. Antikörper und anschließendem chromogenen Test detektiert.

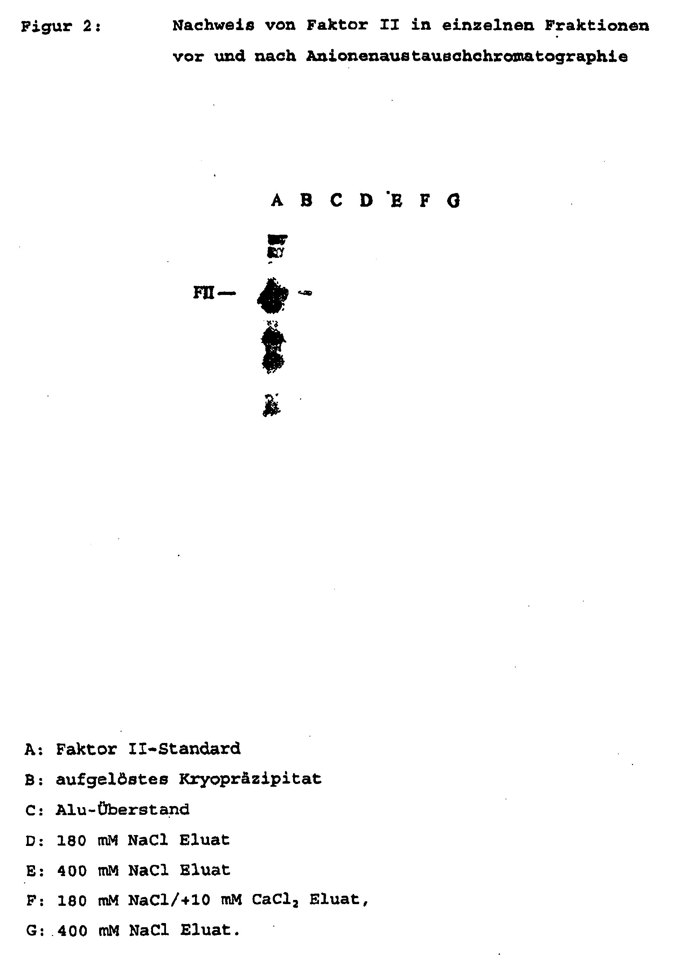

Aus der Figur 2 ist ersichtlich, daß Kryopräzipitat Faktor II enthält. Dieser wird trotz vorangegangener Aluminiumhydroxid-Fällung als Verunreinigung bei 400 mM NaCl (zusammen mit FVIII/vWF) vom Anionenaustauscher eluiert (Spur E). Faktor II kann jedoch durch 10 mM CaCl₂ selektiv eluiert werden (Spur F), und vWF/FVIII bei nachfolgender Elution mit 400 mM NaCl frei von Faktor II gewonnen werden (Spur G).

### b. Nachweis von Protein S in den einzelnen Reinigungsstufen

Zum Nachweis von Protein S in den Reinigungsstufen wurde polyklonales Kaninchen-anti-Protein S-Serum (Assera Protein S, Stago) als 1. Antikörper eingesetzt und mit Ziegen-anti-Kaninchen-IgG-HRP-Konjugat (Fa. Bio-Rad) als 2. Antikörper und anschließendem chromogenen Test detektiert.

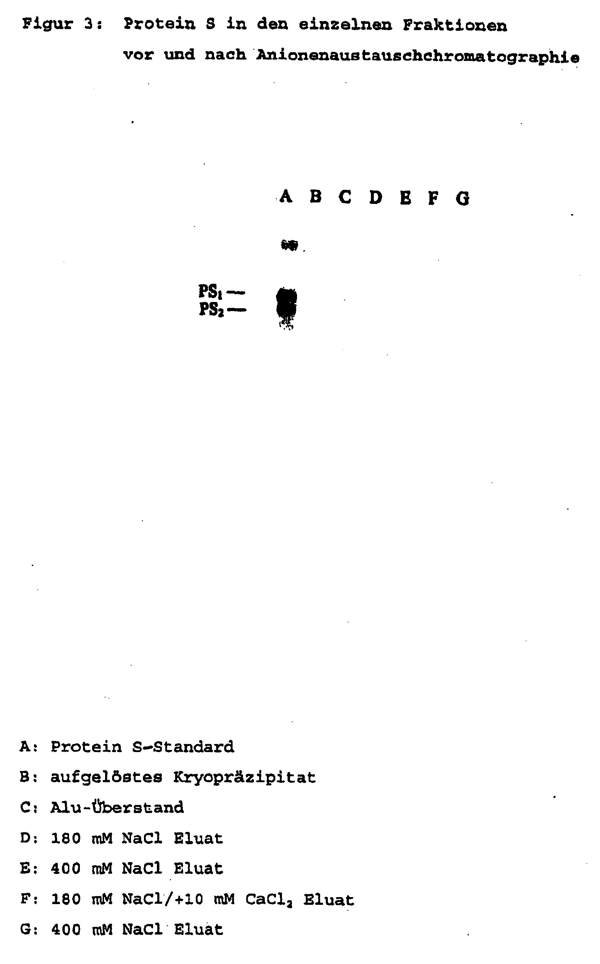

Figur 3 zeigt den Nachweis von Protein S in einzelnen Reinigungsstufen vor und nach der Anionenaustauschchromatographie und nach Entfernen von Protein S durch Calciumchlorid-Elution.

Aus der Figur 3 ist ersichtlich, daß Protein S aus Krybpräzipitat trotz vorheriger Aluminiumhydroxid-Fällung als Verunreinigung bei 400 mM NaCl (zusammen mit FVIII/vWF) vom Anionenaustauscher eluiert (Spur E). Protein S kann jedoch durch 10 mM CaCl₂ selektiv eluiert werden (Spur F), und FVIII/vWF bei nachfolgender Elution mit 400 mM NaCl frei von Protein S gewonnen werden (Spur G).

### c. Nachweis von Faktor IX in den einzelnen Reinigungsstufen

Zum Nachweis von Faktor IX in den Reinigungsstufen wurde polyklonales Kaninchen-anti-Faktor IX-Serum (Assera Faktor XI, Stago) als 1. Antikörper eingesetzt und mit Ziegen-anti-Kaninchen-IgG-HRP-Konjugat (Fa. Bio-Rad) als 2. Antikörper und anschließendem chromogenen Test detektiert.

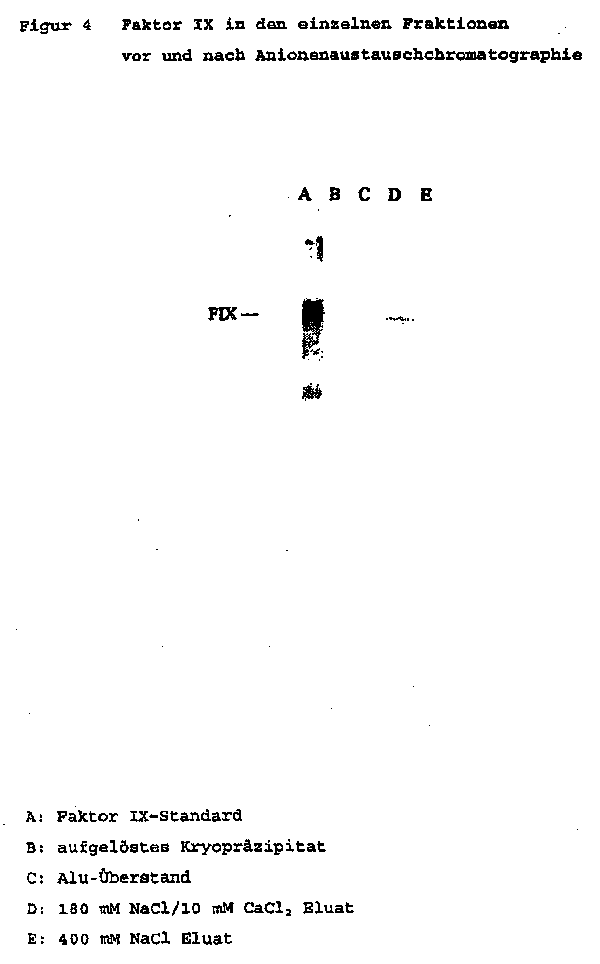

Figur 4 zeigt den Nachweis von Faktor IX in einzelnen Reinigungsstufen vor und nach der Anionenaustauschchromatographie und selektivem Entfernen von Faktor IX durch Calciumchlorid.

Aus der Figur 4 ist ersichtlich, daß Faktor IX trotz vorheriger Aluminiumhydroxid-Fällung als Verunreinigung bei 400 mM NaCl (zusammen mit Faktor VIII-Komplex) vom Anionenaustauscher eluiert. Durch Zugabe von 10 mM CaCl₂ wird Faktor IX jedoch selektiv eluiert (Spur D) und FVIII/vWF bei nachfolgender Elution mit 400 mM NaCl frei von Faktor IX gewonnen (Spur E).

### Beispiel 3:

### Entfernen von Plasmaproteasen und Gewinnung von hochreinem Faktor VIII/vWF-Komplex

Ziel der Untersuchungen war es, ein vWF/FVIII-Präparat zu gewinnen, frei von Proteasen und anderen Gerinnungsfaktoren. Eine wesentliche Verunreinigung des Kryopräzipitates stellt die Protease Plasminogen dar. Diese findet sich auch im FVIII/vWF-Eluat (400 mM Eluat) wieder.

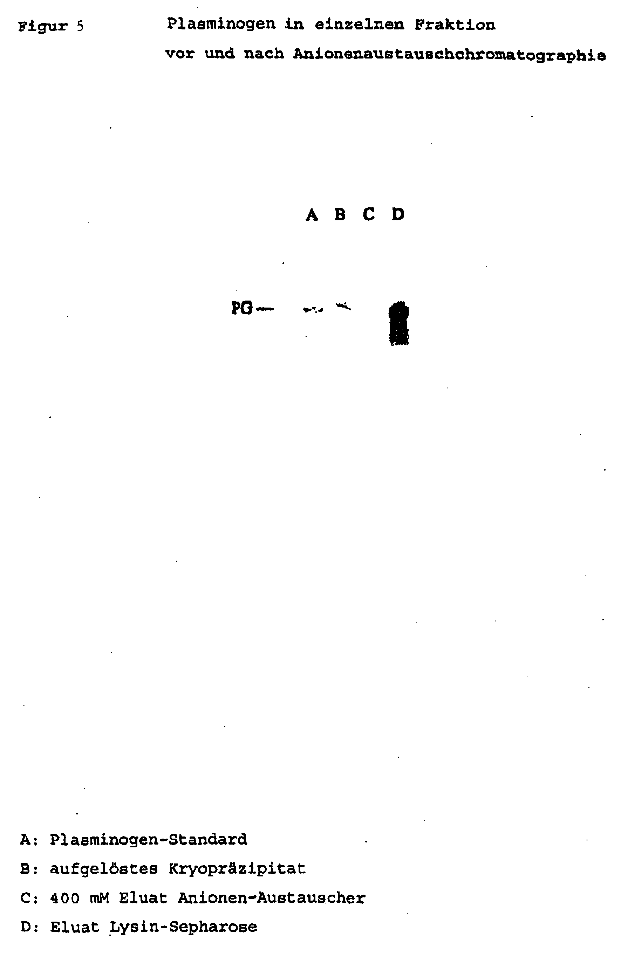

Zur Entfernung von Plasminogen wurde Kryopräzipitat, wie oben beschrieben, aufgelöst und mit Aluminiumhydroxidgel behandelt. Anschließend wurde der Alu-Überstand durch ein Lysin-Sepharosegel filtriert, und davon direkt auf den Anionenaustauscher (Fractogel EMD TMAE) aufgetragen. FVIII/vWF wurde, wie beschrieben, vom Anionenaustauscher mit 400 mM NaCl eluiert. Die Eluate vor und nach der Anionenaustauschchromatographie wurden auf Plasminogen mittels Westernblot analysiert (Figur 5). Dazu wurden die Proteine mittels SDS-PAGE gelelektrophoretisch aufgetrennt, auf eine Membran geblottet und Plasminogen mit einem polyklonalen Kaninchen-anti-Plasminogen-Serum (Stago) als 1. Antikörper und anschließendem chromogenen Test detektiert.

Aus den Ergebnissen ist ersichtlich, daß durch Filtration an Lysin-Sepharose die Protease Plasminogen vom vWF/FVIII selektiv abgetrennt wird.

### Beispiel 4: (derzeit nach Ansicht der Anmelderin der beste Weg zur Ausführung der Erfindung)

### Heparin-Affinitätschromatographie von FVIII/vWF-Komplex

Für die Heparin-Affinitätschromatographie wurde Fractogel AF EMD-Heparin verwendet. Als Ausgangsmaterial diente FVIII/vWF, der durch Anionenaustauschchromatographie gemäß Beispiel 2 gereinigt wurde (400 mM Eluat). Zur Reinigung von FVIII/vWF über Affinitätschromatographie wurden 27 ml des 400 mM NaCl-Fractogel-Eluats 4-fach mit 81 ml Tris-HCl-Puffer (pH 7,4) verdünnt und auf die Heparin-Affinitätssäule aufgetragen. Die Säule wurde zuerst mit 100 mM NaCl und anschließend zum Entfernen unspezifisch gebundener Proteine mit 160 mM NaCl gewaschen. Der Faktor VIII/vWF-Komplex wurde anschließend durch Elution der Heparin-Säule mit 300 mM NaCl erhalten. Die Faktor VIII:C- und vWF:RistoCoF-Aktivität sowie der vWF:Ag-Gehalt im Ausgangsmaterial und den einzelnen Fraktionen der Anionenaustauschchromatographie und der Heparin-Affinitätschromatographie wurden bestimmt und sind in den Tabellen 4 A und 4 B zusammengefaßt.

**Tabelle 4 A:**

| **Faktor VIII:C- und vWF:RistoCoF-Aktivität des Ausgangsmaterials und der einzelnen Fraktionen vor und nach Anionenaustauschchromatographie** | | | | |
|---|---|---|---|---|
| Probe | Volumen | vWF:Ag | vWP:RistCoF | FVIII:C |
| | (ml) | (µg/ml) | (mU/ml) | (mU/ml) |
| Alu-Überstand | 75 | 55 | 1700 | 5260 |
| 180 mM Eluat/ 10 mM CaCl₂ | 64 | 21 | 43 | - |
| 200 mM Eluat | 33 | 1 | - | - |
| 400 mM Eluat | 29 | 137 | 4250 | 9500 |

**Tabelle 4 B:**

| **Faktor VIII:C- und vWF:RistoCoF-Aktivität des Ausgangsmaterials und der einzelnen Fraktionen vor und nach Heparin-Affinitätschromatographie** | | | | |
|---|---|---|---|---|
| Probe | Volumen | vWF:Ag | vWF:RistCoF | FVIII:C |
| | (ml) | (µg/ml) | (mU/ml) | (mU/ml) |
| Ausgangsmaterial | 104 | 42 | 850 | 2630 |
| 160 mM Eluat | 42 | 11 | 43 | 650 |
| 300 mM Eluat | 28 | 92 | 2550 | 5840 |

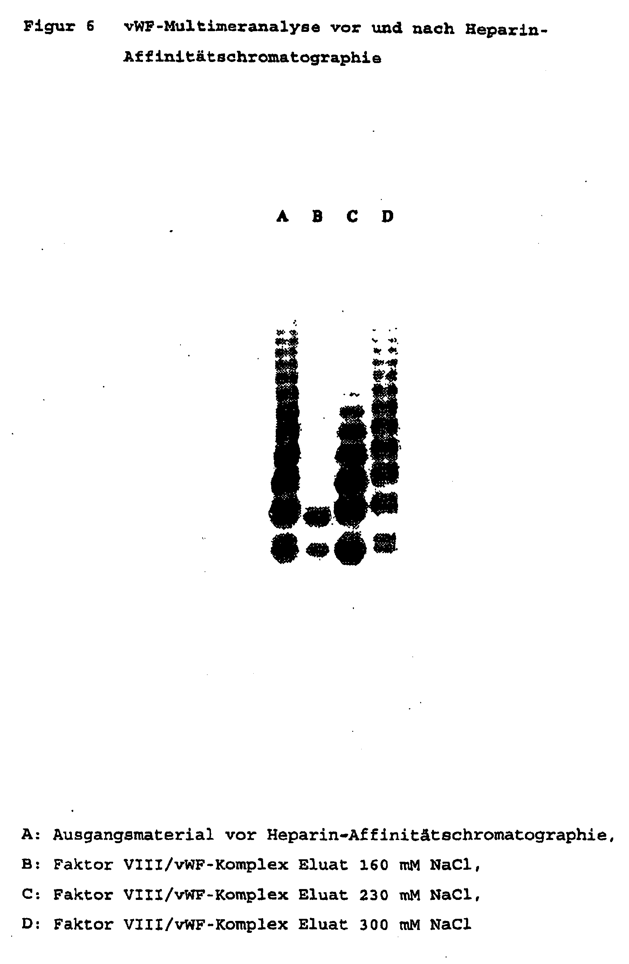

Die Eluate der Heparin-Affinitätschromatographie wurden auf vWF-Polymerzusammensetzung untersucht (Figur 6).

Aus der Figur 6 ist ersichtlich, daß der hochmolekulare Anteil des vWF in der 300 mM NaCl-Fraktion erhalten wird. Diese Fraktion besitzt auch die höchste Faktor VIII:C- und vWF-Ristocetin-Cofaktoraktivität (Tabelle 4 B).

Aus der Summe der Ergebnisse ist klar erkennbar, daß sich durch die Kombination aus Anionenaustauschchromatographie und Heparin-Affinitätschromatographie sowohl vWF, FVIII, als auch deren Komplex aus Kryopräzipitat isolieren und reinigen lassen. Insbesondere mittels Heparin-Affinitätschromatographie ist es möglich, niedermolekulare vWF-Multimere und Abbauprodukte des vWF aus Kryopräzipitat abzutrennen.

### Beispiel 5:

### Bestimmung der spezifischen Ristocetin-CoFaktor-Aktivität von gereinigtem vWF oder vWF-Komplex

Mittels chromatographischer Methoden wurde plasmatischer vWF (p-vWF) aus humanem Kryopräzipitat und rekombinanter vWF (rvWF) aus dem Fermentationsüberstand rekombinanter CHO-Zellen isoliert und gemäß Beispiel 2 gereinigt. Durch Heparin-Affinitätschromatographie und Elution mit unterschiedlichen Salzkonzentrationen wurden Fraktionen mit unterschiedlichem Polymerisationsgrad an vWF isoliert (gemäß Beispiel 4). Insgesamt wurden für p-vWF- und r-vWF-Fraktionen mit low-molecular-weight vWF (vWF/LMW) bei 120 mM NaCl, mit medium molecular weight (vWF/MMW) bei 230 mM NaCl und high molecular weight (vWF/HMW) bei 300 mM NaCl erhalten. Diese Fraktionen wurden auf ihren Gehalt an vWF:Ag mittels ELISA (Asserachrom vWF® , Boehringer Mannheim), an Ristocetin-Cofactor-Aktivität (vWF Reagenz, Behringwerke), ihre Multimerstruktur mittels SDS-PAGE und ihre Plättchenbindung untersucht.

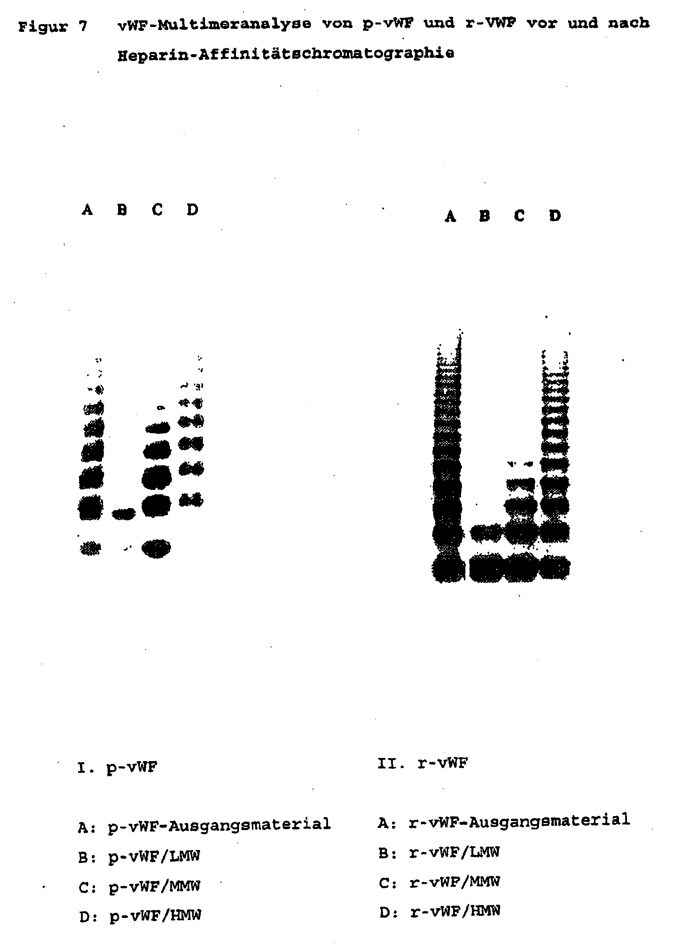

Figur 7 zeigt die vWF-Polymeranalyse von plasmatischem vWF und rekombinantem vWF.

Besonders auffällig ist, daß vor der Reinigung von plasmatischem vWF im Kryopäzipitat die vWF-Dimere, -Tetramere oder -Multimere als Triplett-Strukturen vorliegen. Diese Triplett-Strukturen sind Abbauprodukte von vWF-Multimeren und sind auf im Plasma vorkommende Protease zurückzuführen. Nach der Reinigung sind insbesondere bei der vWF-MMW und vWF-HMW-Fraktionen nur noch Multimere mit Duplett-Strukturen zu erkennen. Durch das chromatographische Verfahren werden so vWF-Multimere mit veränderter Zusammensetzung und Struktur im Vergleich zu im Plasma vorkommenden vWF-Molekülen erhalten, was auf eine Abreicherung von Proteasen und niedermolekularen vWF-Abbauprodukten zurückzuführen ist.

Gegenüber plasmatischen vWF-Multimeren ist bei rekombinantem vWF deutlich das Vorkommen nur einer Singulett-Bande in den vWF-Multimeren zu erkennen. Die rekombinanten vWF-Multimer-Moleküle weisen eine hohe strukturelle Integrität auf und enthalten keine proteolytischen Abbauprodukte, im Vergleich zu den aus der Literatur bekannten vWF-Triplett-Strukturen des plasmatischen vWF.

Tabelle 5 und Tabelle 6 zeigen die spezifische Ristocetin-Cofactor-Aktivität (RistCoF/vWF:Ag) für p-vWF und r-vW.

**Tabelle 5:**

| **Spezifische Ristocetin-Cofactor Aktivität für verschiedene p-vWF-Fraktionen** | |
|---|---|
| Probe | Spezifische RistCoF-Aktivität (mU RistCoF / vWF:Ag) |
| p-vWP/LMW | 3 |
| p-vWF/MMW | 10 |
| p-vWF/HMW | 56 |

**Tabelle 6:**

| **Spezifische Ristocetin-Cofactor-Aktivität für verschiedene r-vWF-Fraktionen** | |
|---|---|
| Probe | Spezifische RistCoF-Aktivität (mU RistCoF / vWF:Ag) |
| r-vWP/LMW | 1 |
| r-vWF/MMW | 6 |
| r-vWF/HMW | 41 |

### Beispiel 6: Bindung von p-vWF und r-vWF an Plättchen

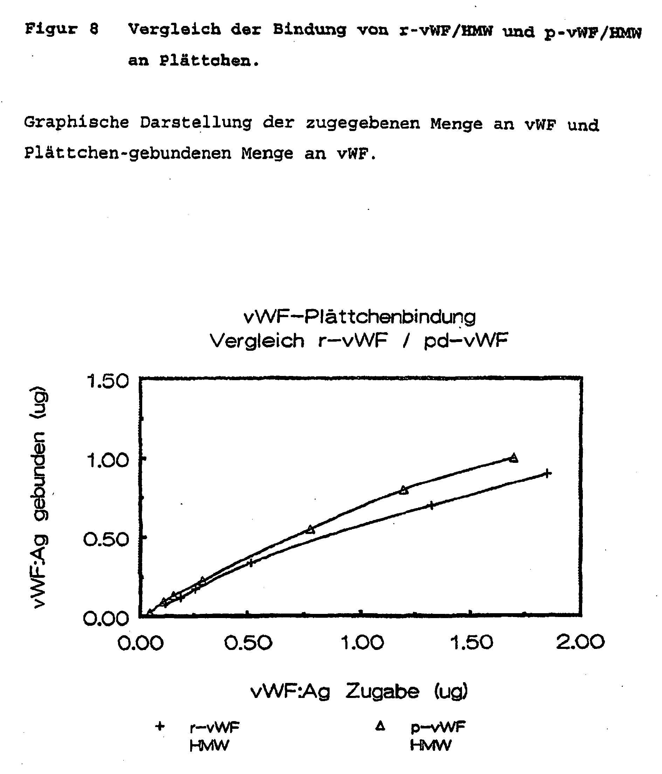

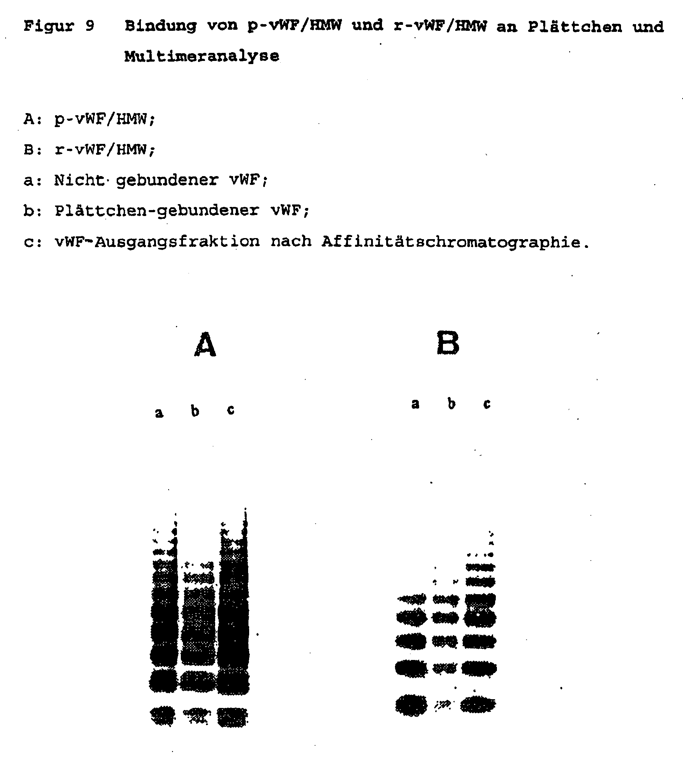

In einer weiteren Untersuchung wurde die Bindung von p-vWF und r-vWF an Plättchen untersucht. p-vWF/HMW bzw. r-vWF/HMW wurden mit konstanter Konzentration an Plättchen und Ristocetin inkubiert. Anschließend wurden die Plättchen durch Zentrifugation abgetrennt (Plättchensediment, gebundener vWF) und ein Überstand (nicht gebundener vWF) erhalten. Im Ausgangsmaterial und im Überstand wurde vWF:Ag und die Plättchen-gebundene Menge an vWF bestimmt. Als Kontrolle wurden idente Inkubationen ohne Ristocetin durchgeführt. Diese ergaben keine vWF-Plättchenbindungen. Das Verhältnis aus vWF-Konzentration im Inkubationsansatz und Plättchen-gebundenem vWF ist in Figur 8 dargestellt und zeigt im direkten Vergleich das Bindungsverhalten von p-vWF/HMW und r-vWF/HMW. Anschließend an die Inkubationen wurden sowohl die Überstände (nicht gebundener vWF) als auch vWF im Plättchensediment (gebundener vWF) auf Multimerzusammensetzung untersucht. Die Ergebnisse der Multimeranalyse sind in Figur 9 zusammengestellt.

### Beispiel 7:

### Bestimmung der Stabilität von gereinigtem Faktor VIII/vWF-Komplex

Durch Anionenaustausch- bzw. Heparin-Affiniätschromatographie erhaltene Fraktionen wurden auf die Stabilität der vWF-Multimere sowie der Faktor VIII-Aktivität untersucht. Dazu wurden die in den einzelnen Reinigungschritten erhaltenen Fraktionen bei -20°C, 4°C und Raumtemperatur für eine Zeitdauer bis zu 60 Tagen gelagert und jeweils nach 0, 1, 3, 5, 10, 15, 20, 30 und 60 Tagen Proben einer vWF-Multimeranalyse, einer Faktor VIII:C- und vWF-Ristocetin-Cofaktor-Aktivitätsbestimmung unterzogen. Die Eluate der Anionenaustauschchromatographie bzw. Heparin-Affinitätschromatographie, bei denen durch Lysin-Sepharose die Plasmaprotease bzw. durch Calciumchlorid-Elution die Vitamin K-abhängigen Faktoren selektiv entfernt worden waren, zeigten dabei die größte Stabilität. Das vWF-Multimermuster war in diesen Proben auch nach 30 Tagen unverändert, während in den Proben, die keiner Lysin-Sepharose-Chromatographie bzw. Calciumchlorid-Elution unterzogen worden waren, abhängig von der Zeitdauer ein Auftreten von proteolytischen vWF-Abbauprodukten zu erkennen war. Insbesondere ist daher für die Erhaltung der Stabilität der hochmolekularen vWF-Multimeren die Entfernung von im Ausgangsmaterial vorhandenen Plasmaproteinen notwendig, da diese die Lagerstabilität stark beeinflußen bzw. herabsetzen.

### Beispiel 8:

### Erhöhung der Stabilität des gereinigten Faktor VIII-Komplexes durch Zugabe von gereinigten hochmolekularen vWF-Multimeren

Zu verschiedenen durch chromatographische Reinigungsschritte erhaltenen Faktor VIII- bzw. Faktor VIII/vWF-Komplex-haltigen Fraktionen wurden unterschiedliche Mengen an gereinigtem p-vWF/HMW oder r-vWF/HMW zugegeben, die Mischungen bei 4°C und Raumtemperatur über einen Zeitraum bis zu 40 Tagen inkubiert und die vWF-Multimerzusammensetzung sowie die Faktor VIII:C- und vWF-Ristocetin-Cofaktor-Aktivität nach 0, 1, 5, 10, 20, 25, 30, 35 und 40 Tagen bestimmt. Die Stabilität der vWF-Multimere sowie die spezifische Ristocetin-CoFaktor-Aktivität war bei Eluaten, bei denen durch vorangegangene Chromatographie Plasmaproteine, insbesondere Plasmaproteasen, entfernt worden waren, am besten. Durch die Zugabe einer vWF/HMW-haltigen Fraktion zu den einzelnen Faktor VIII- bzw. Faktor VIII/vWF-haltigen Fraktionen bzw. zum Ausgangsmaterial aus Kryopräzipitat konnte insbesondere in den Fraktionen, die gemäß Beispiel 7 eine geringere Stabilität aufwiesen, eine Verbesserung der Stabilität erreicht werden. Abhängig von der Zugabe der Menge an hochmolekularen vWF-Multimeren konnte das Auftreten von proteolytischen Abbauprodukten sowie eine Reduktion der spezifische Aktivität an Faktor VIII und vWF-Aktivität zeitlich hinausgezögert werden.

### Beispiel 9:

### Virusinaktivierung von gereinigtem Faktor VIII/vWF-Komplex bzw. gereinigtem Faktor VIII/vWF-Komplex nach Zugabe von hochmolekularen vWF-Multimeren und Bestimmung der Faktor VIII:C- und vWF-RistoCoF-Aktivität

Einzelne Fraktionen der chromatographischen Reinigungsschritte, sowie Fraktionen, zu denen gereinigte hochmolekulare vWF-Multimere bzw. Albumin zugegeben wurden, wurden einem Virusinaktivierungsverfahren unterzogen. Dazu wurden die Proben für 10 Stunden auf 60°C erhitzt und anschließend für 1 Stunde nochmals bei 80°C weiterinkubiert. Anschließend wurde eine vWF-Multimer-Analyse und eine Bestimmung der Aktivität der Faktor VIII:C- und spezifischen vWF-Plättchenagglutinationsaktivität durchgeführt. Es zeigte sich, daß insbesondere Proben, die nach Heparin-Affinitätschromatographie einen besonders hohen Anteil an hochmolekularen vWF-Multimeren und keine niedermolekularen vWF-Abbauprodukte enthalten, sowie eine hohe spezifische Ristocetin-Cofaktor-Aktivität aufwiesen, den geringsten Aktivitätsverlust an Faktor VIII und vWF zeigten. Auch bei Proben, denen zusätzlich noch eine bestimmte Menge an gereinigten hochmolekularen vWF-Multimeren zugesetzt wurde, war der Aktivitätsverlust nach dem Inaktivierungsverfahren maximal 10%. Bei den Proben, denen Albumin zugesetzt worden war, sank die spezifische Aktivität bei Zugabe des Stabilisators und dann nochmals nach dem Inaktivierungsverfahren. Dadurch konnte gezeigt werden, daß durch die Anwesenheit von ausschließlich vWF/HMW bzw. durch Zugabe von hochmolekularen vWF-Multimeren die Stabilität der Proteine im Faktor VIII/vWF-Komplex wesentlich erhöht werden kann, ohne daß die spezifische Aktivität wesentlich reduziert wird.

## Patentansprüche

1. Stabiles, virussicheres Faktor VIII-Komplex-Konzentrat, enthaltend insbesondere hochmolekulare vWF-Multimere mit hoher struktureller Integrität, wobei die vWF-Multimeren aus einer Singulett- oder einer Duplett-Struktur bestehen und frei sind von proteolytischen Abbauprodukten des vWF.

2. Stabiles, virussicheres Faktor VIII-Komplex-Konzentrat nach Anspruch 1, **dadurch gekennzeichnet, daß** es eine spezifische Plättchenagglutinationsaktivität von mindestens 50 U/mg vWF:Ag aufweist.

3. Stabiles, virussicheres Faktor VIII-Komplex-Konzentrat nach einem der Ansprüche 1 oder 2, **dadurch gekennzeichnet, daß** es ein molares Verhältnis von Faktor VIII zu vWF zwischen 0,01 und 100 aufweist.

4. Stabiles, virussicheres Faktor VIII/vWF-Komplex-Konzentrat nach Anspruch 3, **dadurch gekennzeichnet, daß** das molare Verhältnis von Faktor VIII zu vWF zwischen 0,05 und 1 beträgt.

5. Stabiles, virussicheres Faktor VIII-Komplex-Konzentrat nach einem der Ansprüche 1 bis 4, **dadurch gekennzeichnet, daß** es frei ist von Plasmaproteinen, insbesondere von Plasmaproteasen, und frei von mikrobiologischen und molekularbiologischen Pathogenen ist.

6. Pharmazeutische Zusammensetzung, enthaltend einen stabilen Faktor VIII/vWF-Komplex nach einem der Ansprüche 1 bis 5.

7. Pharmazeutische Zusammensetzung nach Anspruch 6, **dadurch gekennzeichnet, daß** sie einen physiologisch akzeptablen Träger enthält.

8. Verwendung eines stabilen Faktor VIII/vWF-Komplexes nach einem der Ansprüche 1 bis 5 zur Herstellung eines Mittels zur Behandlung von Hämophile A und/oder verschiedener Formen des von Willebrand-Syndroms.

9. Verfahren zur Gewinnung von stabilem Faktor VIII/vWF-Komplex, **dadurch gekennzeichnet, daß** Faktor VIII/vWF-Komplex aus einer Proteinlösung an einen Heparin-Affinitätsträger gebunden wird und Faktor VIII/vWF-Komplex bei einer Salzkonzentration zwischen ≥ 200 und ≤ 300 mM gewonnen wird.

10. Verfahren nach Anspruch 9, **dadurch gekennzeichnet, daß** als Proteinlösung eine Plasmafraktion oder ein Kryopräzipitat, enthaltend Faktor VIII/vWF-Komplex, verwendet wird.

11. Verfahren nach Anspruch 9, **dadurch gekennzeichnet, daß** als Proteinlösung ein zellfreier Kulturüberstand von transformierten Zellen, der Faktor VIII/vWF-Komplex enthält, zum Einsatz kommt.

12. Verfahren nach Anspruch 9, **dadurch gekennzeichnet, daß** als Proteinlösung eine angereicherte Proteinfraktion verwendet wird.

13. Verfahren nach einem der Ansprüche 9 bis 12, **dadurch gekennzeichnet, daß** als Affinitätsträger ein Träger mit daran gebundenem Heparin verwendet wird, vorzugsweise AF-Heparin Toyopearl® (Tosohaas), Heparin EMD-Fraktogel® oder Heparin-Sepharose Fast Flow® .

14. Verfahren nach einem der Ansprüche 9 bis 13, **dadurch gekennzeichnet, daß** als Puffersystem für die Affinitätschromatographie eine Pufferlösung bestehend aus Puffersubstanzen, vorzugsweise Tris/HCl-Puffer, Phosphatpuffer oder Citratpuffer und gegebenenfalls Salz verwendet wird.

15. Verfahren nach einem der Ansprüche 9 bis 14, **dadurch gekennzeichnet, daß** die Affinitätschromatographie in einem pH-Bereich von 6,0 bis 8,5, vorzugsweise bei einem pH-Wert von 7,4, erfolgt.

16. Verfahren nach einem der Ansprüche 9 bis 15, **dadurch gekennzeichnet, daß** als Salz NaCl verwendet wird.

17. Verfahren nach einem der Ansprüche 9 bis 16, **dadurch gekennzeichnet, daß** ein Faktor VIII/VWF-Komplex mit einer spezifischen Aktivität von Faktor VIII:C von mindestens 50 U/mg Protein und einer spezifischen Plättchenagglutinationsaktivität von mindestens 50 U/mg vWF erhalten wird.

18. Verfahren zur Gewinnung von stabilem Faktor VIII/vWF-Komplex, bei dem Faktor VIII/vWF-Komplex aus einer unreinen Proteinlösung an einen Anionenaustauscher gebunden wird, **dadurch gekennzeichnet, daß** kontaminierende Plasmaproteine bei einer Salzkonzentration von ≤ 200 mM mit CaCl₂ selektiv eluiert werden und anschließend Faktor VIII/vWF-Komplex vom Anionenaustauscher mit einer Salzkonzentration zwischen ≥ 200 und ≤ 400 mM erhalten wird.

19. Verfahren nach Anspruch 18, **dadurch gekennzeichnet, daß** die kontaminierenden Proteine Plasmaproteine sind.

20. Verfahren nach Anspruch 18, **dadurch gekennzeichnet, daß** die Plasmaproteine insbesondere Vitamin K-abhängige Faktoren, Plasmaproteasen, Fibronektin oder Fibrinogen sind.

21. Verfahren nach einem der Ansprüche 18 bis 20, **dadurch gekennzeichnet, daß** das CaCl₂ im Elutionsmittel in einer Konzentration zwischen 1 mM und 15 mM, vorzugsweise von 10 mM, verwendet wird.

22. Verfahren nach einem der Ansprüche 18 bis 21, **dadurch gekennzeichnet, daß** die Elution in einem pH-Bereich von 6,0 bis 8,5, vorzugsweise bei einem pH-Wert von 7,4, erfolgt.

23. Verfahren nach einem der Ansprüche 18 bis 22, **dadurch gekennzeichnet, daß** als Salz NaCl verwendet wird.

24. Verfahren nach einem der Ansprüche 18 bis 23, **dadurch gekennzeichnet, daß** die Fraktion, enthaltend den gewonnenen Faktor VIII/vWF-Komplex, einem weiteren chromatographischen Schritt, vorzugsweise einer Affinitätschromatographie, unterzogen wird.

25. Verfahren nach Anspruch 24, **dadurch gekennzeichnet, daß** als Affinitätschromatographie eine Heparin-Chromatographie gemäß einem der Ansprüche 9 bis 17 ist.

26. Verfahren zur Herstellung eines stabilen Faktor VIII/vWF-Komplexes, **dadurch gekennzeichnet, daß** einem über ein chromatographisches Verfahren gereinigten Faktor VIII- oder Faktor VIII-Komplex eine gereinigte hochmolekulare Fraktion von vWF-Molekülen zugesetzt wird, wodurch ein Faktor VIII/vWF-Komplex mit einem molaren Verhältnis von Faktor VIII zu vWF zwischen 0,01 und 100, vorzugsweise zwischen 0,05 und 1, erhalten wird.

27. Verfahren nach Anspruch 26, **dadurch gekennzeichnet, daß** der gereinigte Faktor VIII- oder Faktor VIII-Komplex aus einer Plasmafraktion gewonnen wird.

28. Verfahren nach Anspruch 26, **dadurch gekennzeichnet, daß** der gereinigte Faktor VIII- oder Faktor VIII-Komplex aus einem zellfreien Zellkulturüberstand von transformierten Zellen gewonnen wird.

29. Verfahren nach Anspruch 26, **dadurch gekennzeichnet, daß** die gereinigte hochmolekulare Fraktion von vWF-Molekülen aus plasmatischem vWF besteht.

30. Verfahren nach Anspruch 26, **dadurch gekennzeichnet, daß** die gereinigte hochmolekulare Fraktion von vWF-Molekülen aus rekombinantem vWF besteht.

31. Verfahren nach einem der Ansprüche 26 bis 30, **dadurch gekennzeichnet, daß** eine hochmolekulare Fraktion von vWF-Molekülen mit einer spezifischen Plättchenagglutinationsaktivität von mindestens 50 U/mg vWF:Ag gewonnen wird.

## Claims

1. A stable, virus-safe factor VIII-complex concentrate, particularly comprising high molecular vWF multimers of high structural integrity, the vWF multimers being comprised of a singlet or doublet structure and being free from proteolytic degradation products of vWF.

2. A stable, virus-safe factor VIII-complex concentrate according to claim 1, **characterized in that** it has a specific platelet agglutination activity of at least 50 U/mg vWF:Ag.

3. A stable, virus-safe factor VIII-complex concentrate according to claim 1 or 2, **characterized in that** it has a molar ratio of factor VIII to vWF of between 0.01 and 100.

4. A stable, virus-safe factor VIII/vWF-complex concentrate according to claim 3, **characterized in that** the molar ratio of factor VIII to vWF is between 0.05 and 1.

5. A stable, virus-safe factor VIII-complex concentrate according to any one of claims 1 to 4, **characterized in that** it is free from plasma proteins, in particular plasma proteases, and free from microbiological and molecular-biological pathogens.

6. A pharmaceutical composition containing a stable factor VIII/vWF-complex according to any one of claims 1 to 5.

7. A pharmaceutical composition according to claim 6, **characterized in that** it comprises a physiologically acceptable carrier.

8. The use of a stable factor VIII/vWF-complex according to any one of claims 1 to 5 for producing an agent for treating hemophilia A and/or various forms of von Willebrand syndrome.

9. A method of recovering stable factor VIII/vWF-complex, **characterized in that** factor VIII/vWF-complex from a protein solution is bound to a heparin affinity carrier and factor VIII/ vWF-complex is recovered at a salt concentration of between ≥ 200 and ≤ 300 mM.

10. A method according to claim 9, **characterized in that** a plasma fraction or a cryoprecipitate comprising factor VIII/vWF-complex is used as the protein solution.

11. A method according to claim 9, **characterized in that** a culture supernatant from transformed cells which is free from cells and comprises factor VIII/vWF-complex is used as the protein solution.

12. A method according to claim 9, **characterized in that** an enriched protein fraction is used as the protein solution.

13. A method according to any one of claims 9 to 12, **characterized in that** a carrier with heparin bound thereto, preferably AF-Heparin Toyopearl® (Tosohaas), Heparin EMD-Fraktogel® or Heparin-Sepharose Fast Flow®, is used as affinity carrier.

14. A method according to any one of claims 9 to 13, **characterized in that** a buffer solution comprised of buffer substances, preferably Tris/HCl buffer, phosphate buffer or citrated buffer and, optionally, salt is used as the buffer system for the affinity chromatography.

15. A method according to any one of claims 9 to 14, **characterized in that** the affinity chromatography is effected in a pH range of from 6.0 to 8.5, preferably at a pH of 7.4.

16. A method according to any one of claims 9 to 15, **characterized in that** NaCl is used as salt.

17. A method according to any one of claims 9 to 16, **characterized in that** a factor VIII/vTnlF-complex is obtained with a specific activity of factor VIII:C of at least 50 U/mg protein and a specific platelet agglutination activity of at least 50 U/ mg vWF.

18. A method of recovering stable factor VIII/vWF-complex, wherein factor VIII/vWF-complex from a contaminated protein solution is bound to an anion exchanger, **characterized in that** contaminating plasma proteins are selectively eluted with CaCl₂ at a salt concentration of ≤ 200 mM, and subsequently factor VIII/vWF-complex is obtained from the anion exchanger with a salt concentration of between ≥ 200 and ≤ 400 mM.

19. A method according to claim 18, **characterized in that** the contaminating proteins are plasma proteins.

20. A method according to claim 19, **characterized in that** the plasma proteins particularly are vitamin K-dependent factors, plasma proteases, fibronectin or fibrinogen.

21. A method according to any one of claims 18 to 20, **characterized in that** the CaCl₂ is used in the eluting agent at a concentration of between 1 mM and 15 mM, preferably 10 mM.

22. A method according to any one of claims 18 to 21, **characterized in that** the elution is effected in a pH range of from 6.0 to 8.5, preferably at a pH of 7.4.

23. A method according to any one of claims 18 to 22, **characterized in that** NaCl is used as the salt.

24. A method according to any one of claims 18 to 23, **characterized in that** the fraction comprising the recovered factor VIII/vWF-complex is subjected to a further chromatographic step, preferably to an affinity chromatography.

25. A method according to claim 24, **characterized in that** a heparin chromatography according to any one of claims 9 to 17 is used as the affinity chromatography.

26. A method of producing a stable factor VIII/vWF complex, **characterized in that** a purified high-molecular fraction of vWF molecules is admixed to a factor VIII or factor VIII/vWF-complex which has been purified via a chromatographic method, a factor VIII/vWF-complex having a molar ratio of factor VIII to vWF of between 0.01 and 100, preferably of between 0.05 and 1, being obtained thereby.

27. A method according to claim 26, **characterized in that** the purified factor VIII or factor VIII/vWF-complex is recovered from a plasma fraction.

28. A method according to claim 26, **characterized in that** the purified factor VIII or factor VIII/vWF-complex is recovered from a cell culture supernatant from transformed cells which is free from cells.

29. A method according to claim 26, **characterized in that** the purified high-molecular fraction of vWF molecules is comprised of plasmatic vWF.

30. A method according to claim 26, **characterized in that** the purified high-molecular fraction of vWF molecules is comprised of recombinant vWF.

31. A method according to any one of claims 26 to 30, **characterized in that** a high-molecular fraction of vWF molecules having a specific platelet agglutination activity of at least 50 U/ mg vWF:Ag is recovered.

## Revendications

1. Concentré d'un complexe du facteur VIII, stable, sans virus, contenant en particulier des multimères du vWF de poids moléculaire élevé avec une intégrité de structure élevée, où les multimères du vWF consistent en une structure singulet ou une structure doublet et sont exempts de produits de dégradation protéolytique du vWF.

2. Concentré d'un complexe du facteur VIII, stable, sans virus, selon la revendication 1, **caractérisé en ce qu'**il présente une activité spécifique d'agglutination des plaquettes d'au moins 50 U/mg vWF:Ag.

3. Concentré d'un complexe du facteur VIII, stable, sans virus, selon la revendication 1 ou 2, **caractérisé en ce qu'**il présente un rapport molaire du facteur VIII au vWF allant de 0,01 à 100.

4. Concentré d'un complexe du facteur VIII/vWF, stable, sans virus, selon la revendication 3, **caractérisé en ce que** le rapport molaire du facteur VIII au vWF se situe dans l'intervalle allant de 0,05 à 1.

5. Concentré d'un complexe du facteur VIII, stable, sans virus, selon l'une quelconque des revendications 1 à 4, **caractérisé en ce qu'**il est exempt de protéines plasmatiques, en particulier de protéases plasmatiques et exempt de pathogènes microbiologiques et de biologie moléculaire.

6. Composition pharmaceutique contenant un complexe facteur VIII/vWF stable selon l'une quelconque des revendications 1 à 5.

7. Composition pharmaceutique selon la revendication 6, **caractérisé en ce qu'**elle contient un support physiologiquement acceptable.

8. Utilisation d'un complexe facteur VIII/vWF stable selon l'une quelconque des revendications 1 à 5, pour la préparation d'un agent pour le traitement de l'hémophilie A et/ou de différentes formes du syndrome de von Willebrand.

9. Procédé d'obtention d'un complexe facteur VIII/vWF stable, **caractérisé en ce que** le complexe facteur VIII/vWF est fixé sur un support d'affinité à l'héparine depuis une solution protéique et que le complexe facteur VIII/vWF est obtenu pour une concentration saline allant de ≥ 200 à ≤ 300 mM.

10. Procédé selon la revendication 9, **caractérisé en ce que** l'on utilise comme solution protéique, une fraction plasmatique ou un cryoprécipité, contenant le complexe facteur VIII/vWF.

11. Procédé selon la revendication 9, **caractérisé en ce que** l'on met en oeuvre comme solution protéique, un surnageant de culture de cellules transformées, exempt de cellule, qui contient le complexe facteur VIII/vWF.

12. Procédé selon la revendication 9, **caractérisé en ce que** l'on utilise comme solution protéique, une fraction protéique enrichie.

13. Procédé selon l'une quelconque des revendications 9 à 12, **caractérisé en ce que** l'on utilise comme support d'affinité, un support avec de l'héparine liée, de préférence le AF-Heparin Toyopearl® (Tosohaas), l'Heparin EMD-Fraktogel® ou l'Heparin-Sepharose Fast Flow®.

14. Procédé selon l'une quelconque des revendications 9 à 13, **caractérisé en ce que** l'on utilise comme système tampon pour la chromatographie d'affinité, une solution tampon consistant en des substances tampon, de préférence le tampon Tris/HCl, un tampon phosphate ou un tampon citrate et le cas échéant, des sels.

15. Procédé selon l'une quelconque des revendications 9 à 14, **caractérisé en ce que** la chromatographie d'affinité est réalisée dans un intervalle de pH allant de 6,0 à 8,5, de préférence à un pH de 7,4.

16. Procédé selon l'une quelconque des revendications 9 à 15, **caractérisé en ce que** l'on utilise comme sel, du NaCl.

17. Procédé selon l'une quelconque des revendications 9 à 16, **caractérisé en ce que** l'on obtient un complexe facteur VIII/vWF avec une activité spécifique du facteur VIII:C d'au moins 50 U/mg de protéine et une activité spécifique d'agglutination des plaquettes d'au moins 50 U/mg vWF.

18. Procédé d'obtention d'un complexe facteur VIII/vWF stable, dans lequel le complexe facteur VIII/vWF est lié sur un échangeur d'anions à partir d'une solution protéique non pure, **caractérisé en ce que** l'on élue sélectivement les protéines plasmatiques contaminantes pour une concentration saline de ≤ 200 mM avec du CaCl₂ et ensuite, le complexe facteur VIII/vWF est obtenu de l'échangeur d'anions avec une concentration saline allant de ≥ 200 à ≤ 400 mM.

19. Procédé selon la revendication 18, **caractérisé en ce que** les protéines contaminantes sont des protéines plasmatiques.

20. Procédé selon la revendication 18, **caractérisé en ce que** les protéines plasmatiques sont en particulier, des facteurs dépendant de la vitamine K, des protéases plasmatiques, la fibronectine ou le fibrinogène.

21. Procédé selon l'une quelconque des revendications 18 à 20, **caractérisé en ce que** l'on utilise le CaCl₂ dans le milieu d'élution en une concentration allant de 1 mM à 15 mM, de préférence de 10 mM.

22. Procédé selon l'une quelconque des revendications 18 à 21, **caractérisé en ce que** l'élution est réalisée dans un intervalle de pH allant de 6,0 à 8,5, de préférence à un pH de 7,4.

23. Procédé selon l'une quelconque des revendications 18 à 22, **caractérisé en ce que** l'on utilise du NaCl comme sel.

24. Procédé selon l'une quelconque des revendications 18 à 23, **caractérisé en ce que** l'on soumet la fraction contenant le complexe facteur VIII/vWF obtenu à une autre étape de chromatographie, de préférence une chromatographie d'affinité.

25. Procédé selon la revendication 24, **caractérisé en ce que** la chromatographie d'affinité est une chromatographie à l'héparine selon l'une quelconque des revendications 9 à 17.

26. Procédé de préparation d'un complexe facteur VIII/vWF stable, **caractérisé en ce que** l'on ajoute un facteur VIII ou un complexe du facteur VIII purifié par un procédé chromatographique à la fraction purifiée de poids moléculaire élevé de molécules de vWF, ce par quoi on obtient un complexe facteur VII/vWF avec un rapport molaire du facteur VIII au vWF allant de 0,01 à 100, de préférence de 0,05 à 1.

27. Procédé selon la revendication 26, **caractérisé en ce que** le facteur VIII ou le complexe de facteur VIII purifié est obtenu à partir d'une fraction plasmatique.

28. Procédé selon la revendication 26, **caractérisé en ce que** le facteur VIII ou le complexe de facteur VIII purifié est obtenu à partir d'un surnageant de culture de cellules transformées, exempt de cellule.

29. Procédé selon la revendication 26, **caractérisé en ce que** la fraction purifiée de poids moléculaire élevé de molécules de vWF consiste en du vWF plasmatique.

30. Procédé selon la revendication 26, **caractérisé en ce que** la fraction purifiée de poids moléculaire élevé de molécules de vWF consiste en du vWF recombiné.

31. Procédé selon l'une quelconque des revendications 26 à 30, **caractérisé en ce** l'on obtient une fraction de poids moléculaire élevé de molécules de vWF avec une activité spécifique d'agglutination des plaquettes d'au moins 50 U/mg vWF:Ag.
